# EUROPÄISCHE PATENTSCHRIFT

(11) **EP 2 553 113 B1**
(45) Veröffentlichungstag und Bekanntmachung des Hinweises auf die Patenterteilung: **03.11.2021**
(21) Anmeldenummer: 11710196.4
(22) Anmeldetag: 24.03.2011
(51) Int. Cl.: C12P 13/10

(54) **VERFAHREN ZUR HERSTELLUNG VON L-ORNITHIN UNTER VERWENDUNG VON LYSE ÜBEREXPRIMIERENDEN BAKTERIEN**
PROCESS FOR PRODUCING L-ORNITHINE EMPLOYING LYSE OVEREXPRESSING BACTERIA
PROCÉDÉ DE PRÉPARATION DE L-ORNITHINE EN UTILISANT DES BACTÉRIES SUREXPRIMANT LYSE

(30) Priorität: 30.03.2010 DE 102010003419
(43) Veröffentlichungstag der Anmeldung: 06.02.2013
(73) Patentinhaber: Evonik Operations GmbH, 45128 Essen (DE)
(72) Erfinder: CLAES, Wilfried, 33615 Bielefeld (DE); GERSTMEIR, Robert, 33824 Werther (DE)
(74) Vertreter: Evonik Patent Association
(86) Internationale Anmeldenummer: PCT/EP2011/054541
(87) Internationale Veröffentlichungsnummer: WO 2011/124477

(56) Entgegenhaltungen:
- EP-A2- 1 266 966
- BELLMANN, A. ET AL.: "Expression control and specificity of the basic amino acid exporter LysE of Corynebacterium glutamicum", MICROBIOLOGY, Bd. 147, Nr. 7, Juli 2001 (2001-07), Seiten 1765-1774, XP002662828, in der Anmeldung erwähnt
- GUNJI, Y. & HISASHI, Y.: "Enhancement of l-lysine production in methylotroph Methylophilus methylotrophus by introducing a mutant LysE exporter", JOURNAL OF BIOTECHNOLOGY, Bd. 127, Nr. 1, 9. November 2006 (2006-11-09), Seiten 1-13, XP005754673, in der Anmeldung erwähnt
- VRLJIC, M. ET AL.: "A new type of transporter with a new type of cellular function: L-lysine export from Corynebacterium glutamicum", MOLECULAR MICROBIOLOGY, Bd. 22, Nr. 5, 1996, Seiten 815-826, XP000675494, in der Anmeldung erwähnt
- None

### SEQUENCE LISTING

<110> Evonik Degussa GmbH
<120> Verfahren zur fermentativen Herstellung von L-Ornithin
<130> 200900319
<160> 30
<170> Patentin version 3.3
<210> 1
   <211> 1901
   <212> DNA
   <213> Corynebacterium glutamicum ATCC13032
<220>
   <221> misc_feature
   <222> (930)..(61)
   <223> Komplementärer Strang der Kodierregion des Regulatorgens lysG kodierend für das Aktivatorprotein LysG
<220>
   <221> misc_feature
   <222> (1000)..(1000)
   <223> Transkriptionsstart des lysE-Gens
<220>
   <221> CDS
   <222> (1001)..(1702)
   <223> Kodierregion des lysE-Gens
<400> 1
   cctggcccaa ttcctgcggg cgaagaagtg aaaaaccctg aaccttttca gaagtaacta     60
   aggccgcaat ccctcgattg ctgcatcaac gacggcgtct gtgagtctag ctagagatct    120
   agattccagg cgccatcgtt gccaatacat cggtgtgtca atgggtatct catcgaggag    180
   gatcacttct cctgctttta gcatgggagc agcttgggtt tcgggaagaa gtccccaacc    240
   aaggcctcgg cgaattgcct caccaaaacc ttccgccgac gggacaatgg atacgcgcct    300
   gcgccccaca ggaccatcga cgcgcccgtc caggtcacgg tcttgaagca catctttggg    360
   accgaagcgt aagacgggca tcgcagccca atctagtttc ccatcaacca tgtaggcatc    420
   ccgcaatgag ggggttgcaa tggccaagtg gcgcatggtt ccaagttcta ctacttcaca    480
   tcccgccacg ggattagctt cacgggttac cgctcctaaa acatctccac gccgcagcaa    540
   ggataatgtg tgcgcttcat cttccaagcg cagcgtgagc gttgctccac cccaagaagc    600
   tacctcgttg aacacgggag gaaaccatgt ggatagcgaa tctgcgttga tggcgatggt    660
   taacgggatt tcagcaaggc gtccagatag ttgcgcttta gtttctgctt gcagcaacac    720
   cattttccgc gctgcttgca caaggacttc acccgcttcg gttgctttgg ccggttgggt    780
   gcgcgatacc aacactcgac ccacgtgatg ctcgagagct ttaacgcgct gactcaccgc    840
   cgagggggaa atggaaaggg ctaaggaggc gccttcgaag ctgccttcat caatgattga    900
   gagcaaagtg tccagttgaa tggggttcat gaagctatat taaaccatgt taagaaccaa    960
   tcattttact taagtacttc cataggtcac gatggtgatc atg gaa atc ttc att     1015
                                               Met Glu Ile Phe Ile
                                               1               5
   aca ggt ctg ctt ttg ggg gcc agt ctt tta ctg tcc atc gga ccg cag     1063
   Thr Gly Leu Leu Leu Gly Ala Ser Leu Leu Leu Ser Ile Gly Pro Gln
                   10                  15                  20
   aat gta ctg gtg att aaa caa gga att aag cgc gaa gga ctc att gcg     1111
   Asn Val Leu Val Ile Lys Gln Gly Ile Lys Arg Glu Gly Leu Ile Ala
               25                  30                  35
   gtt ctt ctc gtg tgt tta att tct gac gtc ttt ttg ttc atc gcc ggc     1159
   Val Leu Leu Val Cys Leu Ile Ser Asp Val Phe Leu Phe Ile Ala Gly
           40                  45                  50
   acc ttg ggc gtt gat ctt ttg tcc aat gcc gcg ccg atc gtg ctc gat     1207
   Thr Leu Gly Val Asp Leu Leu Ser Asn Ala Ala Pro Ile Val Leu Asp
       55                  60                  65
   att atg cgc tgg ggt ggc atc gct tac ctg tta tgg ttt gcc gtc atg     1255
   Ile Met Arg Trp Gly Gly Ile Ala Tyr Leu Leu Trp Phe Ala Val Met
   70                  75                  80                  85
   gca gcg aaa gac gcc atg aca aac aag gtg gaa gcg cca cag atc att     1303
   Ala Ala Lys Asp Ala Met Thr Asn Lys Val Glu Ala Pro Gln Ile Ile
                   90                  95                  100
   gaa gaa aca gaa cca acc gtg ccc gat gac acg cct ttg ggc ggt tcg     1351
   Glu Glu Thr Glu Pro Thr Val Pro Asp Asp Thr Pro Leu Gly Gly Ser
               105                 110                 115
   gcg gtg gcc act gac acg cgc aac cgg gtg cgg gtg gag gtg agc gtc     1399
   Ala Val Ala Thr Asp Thr Arg Asn Arg Val Arg Val Glu Val Ser Val
           120                 125                 130
   gat aag cag cgg gtt tgg gta aag ccc atg ttg atg gca atc gtg ctg     1447
   Asp Lys Gln Arg Val Trp Val Lys Pro Met Leu Met Ala Ile Val Leu
       135                 140                 145
   acc tgg ttg aac ccg aat gcg tat ttg gac gcg ttt gtg ttt atc ggc     1495
   Thr Trp Leu Asn Pro Asn Ala Tyr Leu Asp Ala Phe Val Phe Ile Gly
   150                 155                 160                 165
   ggc gtc ggc gcg caa tac ggc gac acc gga cgg tgg att ttc gcc gct     1543
   Gly Val Gly Ala Gln Tyr Gly Asp Thr Gly Arg Trp Ile Phe Ala Ala
                   170                 175                 180
   ggc gcg ttc gcg gca agc ctg atc tgg ttc ccg ctg gtg ggt ttc ggc     1591
   Gly Ala Phe Ala Ala Ser Leu Ile Trp Phe Pro Leu Val Gly Phe Gly
               185                 190                 195
   gca gca gca ttg tca cgc ccg ctg tcc agc ccc aag gtg tgg cgc tgg     1639
   Ala Ala Ala Leu Ser Arg Pro Leu Ser Ser Pro Lys Val Trp Arg Trp
           200                 205                 210
   atc aac gtc gtc gtg gca gtt gtg atg acc gca ttg gcc atc aaa ctg     1687
   Ile Asn Val Val Val Ala Val Val Met Thr Ala Leu Ala Ile Lys Leu
       215                 220                 225
   atg ttg atg ggt tag ttttcgcggg ttttggaatc ggtggccttc gcccaaatgt     1742
   Met Leu Met Gly
   230
   tgatgccggc gtcgtgggaa atctcatcga tcgcctccaa ctcggcgtca gaaaactcca   1802
   agttgttgag tgaatcaagg ctgttgtcca gctgctcaac tgacgaagca ccaatcaatg   1862
   cactggtcac ggtatccgcg ccgtactctc cttgctcgc                          1901
<210> 2
   <211> 233
   <212> PRT
   <213> Corynebacterium glutamicum ATCC13032
<400> 2
   Met Glu Ile Phe Ile Thr Gly Leu Leu Leu Gly Ala Ser Leu Leu Leu
   1               5                   10                  15
   Ser Ile Gly Pro Gln Asn Val Leu Val Ile Lys Gln Gly Ile Lys Arg
               20                  25                  30
   Glu Gly Leu Ile Ala Val Leu Leu Val Cys Leu Ile Ser Asp Val Phe
           35                  40                  45
   Leu Phe Ile Ala Gly Thr Leu Gly Val Asp Leu Leu Ser Asn Ala Ala
       50                  55                  60
   Pro Ile Val Leu Asp Ile Met Arg Trp Gly Gly Ile Ala Tyr Leu Leu
   65                  70                  75                  80
   Trp Phe Ala Val Met Ala Ala Lys Asp Ala Met Thr Asn Lys Val Glu
                   85                  90                  95
   Ala Pro Gln Ile Ile Glu Glu Thr Glu Pro Thr Val Pro Asp Asp Thr
               100                 105                 110
   Pro Leu Gly Gly Ser Ala Val Ala Thr Asp Thr Arg Asn Arg Val Arg
           115                 120                 125
   Val Glu Val Ser Val Asp Lys Gln Arg Val Trp Val Lys Pro Met Leu
       130                 135                 140
   Met Ala Ile Val Leu Thr Trp Leu Asn Pro Asn Ala Tyr Leu Asp Ala
   145                 150                 155                 160
   Phe Val Phe Ile Gly Gly Val Gly Ala Gln Tyr Gly Asp Thr Gly Arg
                   165                 170                 175
   Trp Ile Phe Ala Ala Gly Ala Phe Ala Ala Ser Leu Ile Trp Phe Pro
               180                 185                 190
   Leu Val Gly Phe Gly Ala Ala Ala Leu Ser Arg Pro Leu Ser Ser Pro
           195                 200                 205
   Lys Val Trp Arg Trp Ile Asn Val Val Val Ala Val Val Met Thr Ala
       210                 215                 220
   Leu Ala Ile Lys Leu Met Leu Met Gly
   225                 230
<210> 3
   <211> 761
   <212> DNA
   <213> Corynebacterium glutamicum ATCC13032
<220>
   <221> PCR-lysE-Fragment
   <222> (1)..(761)
<400> 3
   tcgatatcat ggaaatcttc attacaggtc tgcttttggg ggccagtctt ttactgtcca      60
   tcggaccgca gaatgtactg gtgattaaac aaggaattaa gcgcgaagga ctcattgcgg     120
   ttcttctcgt gtgtttaatt tctgacgtct ttttgttcat cgccggcacc ttgggcgttg     180
   atcttttgtc caatgccgcg ccgatcgtgc tcgatattat gcgctggggt ggcatcgctt     240
   acctgttatg gtttgccgtc atggcagcga aagacgccat gacaaacaag gtggaagcgc     300
   cacagatcat tgaagaaaca gaaccaaccg tgcccgatga cacgcctttg ggcggttcgg     360
   cggtggccac tgacacgcgc aaccgggtgc gggtggaggt gagcgtcgat aagcagcggg     420
   tttgggtaaa gcccatgttg atggcaatcg tgctgacctg gttgaacccg aatgcgtatt     480
   tggacgcgtt tgtgtttatc ggcggcgtcg gcgcgcaata cggcgacacc ggacggtgga     540
   ttttcgccgc tggcgcgttc gcggcaagcc tgatctggtt cccgctggtg ggtttcggcg     600
   cagcagcatt gtcacgcccg ctgtccagcc ccaaggtgtg gcgctggatc aacgtcgtcg     660
   tggcagttgt gatgaccgca ttggccatca aactgatgtt gatgggttag ttttcgcggg     720
   ttttggaatc ggtggccttc gcccaaatat tgacctaggc a                         761
<210> 4
   <211> 236
   <212> PRT
   <213> Corynebacterium glutamicum R
<220>
   <221> LysE
   <222> (1)..(236)
<400> 4
   Met Val Ile Met Glu Ile Phe Ile Thr Gly Leu Leu Leu Gly Ala Ser
   1               5                   10                  15
   Leu Leu Leu Ser Ile Gly Pro Gln Asn Val Leu Val Ile Lys Gln Gly
               20                  25                  30
   Ile Lys Arg Glu Gly Leu Ile Ala Val Leu Leu Val Cys Leu Ile Ser
           35                  40                  45
   Asp Val Phe Leu Phe Ile Ala Gly Thr Leu Gly Val Asp Leu Leu Ser
       50                  55                  60
   Asn Ala Ala Pro Ile Val Leu Asp Ile Met Arg Trp Gly Gly Ile Ala
   65                  70                  75                  80
   Tyr Leu Leu Trp Phe Ala Val Met Ala Ala Lys Asp Ala Met Thr Asn
                   85                  90                  95
   Lys Val Glu Ala Pro Gln Ile Ile Glu Glu Thr Glu Pro Thr Val Pro
               100                 105                 110
   Asp Asp Thr Pro Leu Gly Gly Ser Ala Val Ala Thr Asp Thr Arg Asn
           115                 120                 125
   Arg Val Arg Val Glu Val Ser Val Asp Lys Gln Arg Val Trp Val Lys
       130                 135                 140
   Pro Met Leu Met Ala Ile Val Leu Thr Trp Leu Asn Pro Asn Ala Tyr
   145                 150                 155                 160
   Leu Asp Ala Phe Val Phe Ile Gly Gly Val Gly Ala Gln Tyr Gly Asp
                   165                 170                 175
   Thr Gly Arg Trp Ile Phe Ala Ala Gly Ala Phe Ala Ala Ser Leu Ile
               180                 185                 190
   Trp Phe Pro Leu Val Gly Phe Gly Ala Ala Ala Leu Ser Arg Pro Leu
           195                 200                 205
   Ser Ser Pro Lys Val Trp Arg Trp Ile Asn Val Val Val Ala Val Val
       210                 215                 220
   Met Thr Ala Leu Ala Ile Lys Leu Met Leu Met Gly
   225                 230                 235
<210> 5
   <211> 233
   <212> PRT
   <213> Corynebacterium glutamicum ATCC14067
<220>
   <221> LysE
   <222> (1)..(233)
<400> 5
   Met Glu Ile Phe Ile Thr Gly Leu Leu Leu Gly Ala Ser Leu Leu Leu
   1               5                   10                  15
   Ser Ile Gly Pro Gln Asn Val Leu Val Ile Lys Gln Gly Ile Lys Arg
               20                  25                  30
   Glu Gly Leu Ile Ala Val Leu Leu Val Cys Leu Ile Ser Asp Val Phe
           35                  40                  45
   Leu Phe Ile Ala Gly Thr Leu Gly Val Asp Leu Leu Ser Asn Ala Ala
       50                  55                  60
   Pro Ile Val Leu Asp Ile Met Arg Trp Gly Gly Ile Ala Tyr Leu Leu
   65                  70                  75                  80
   Trp Phe Ala Val Met Ala Ala Lys Asp Ala Met Thr Asn Lys Val Glu
                   85                  90                  95
   Ala Pro Gln Ile Ile Glu Glu Thr Glu Pro Thr Val Pro Asp Asp Thr
               100                 105                 110
   Pro Leu Gly Gly Ser Ala Val Ala Thr Asp Thr Arg Asn Arg Val Arg
           115                 120                 125
   Val Glu Val Ser Val Asp Lys Gln Arg Val Trp Val Lys Pro Met Leu
       130                 135                 140
   Met Ala Ile Val Leu Thr Trp Leu Asn Pro Asn Ala Tyr Leu Asp Ala
   145                 150                 155                 160
   Phe Val Phe Ile Gly Gly Val Gly Ala Gln Tyr Gly Asp Thr Gly Arg
                   165                 170                 175
   Trp Ile Phe Ala Ala Gly Ala Phe Ala Ala Ser Leu Ile Trp Phe Pro
               180                 185                 190
   Leu Val Gly Phe Gly Ala Ala Ala Leu Ser Arg Pro Leu Ser Ser Pro
           195                 200                 205
   Lys Val Trp Arg Trp Ile Asn Val Val Val Ala Val Val Met Thr Ala
   210 215 220
   Leu Ala Ile Lys Leu Met Leu Met Gly
   225 230
<210> 6
   <211> 702
   <212> DNA
   <213> Corynebacterium glutamicum ATCC14067
<220>
   <221> lysE-CDS
   <222> (1)..(702)
<400> 6
   atggaaatct tcattacagg tctgcttttg ggggccagtc ttttgctgtc catcggaccg 60
   cagaatgtac tggtgattaa acaaggaatt aagcgcgaag gactcattgc ggttcttctc 120
   gtgtgtttaa tttctgacgt ctttttgttc atcgccggca ccttgggcgt tgatcttttg 180
   tccaatgccg cgccgatcgt gctcgatatt atgcgctggg gtggtatcgc ttacctgtta 240
   tggtttgccg tcatggcagc gaaagacgcc atgacaaaca aggtggaagc gccacagatc 300
   attgaagaaa cagaaccaac cgtgcccgat gacacgcctt tgggcggttc ggcggtggcc 360
   actgacacgc gcaaccgggt gcgggtggag gtgagcgtcg ataagcagcg ggtttgggta 420
   aagcccatgt tgatggcaat cgtgctgacc tggttgaacc cgaatgcgta tttggacgcg 480
   tttgtgttta tcggcggcgt cggcgcgcaa tacggcgaca ccggacggtg gattttcgcc 540
   gctggcgcgt tcgcggcaag cctgatctgg ttcccgctgg tgggtttcgg cgcagcagca 600
   ttgtcacgcc cgctgtccag ccccaaggtg tggcgctgga tcaacgtcgt cgtggcagtt 660
   gtgatgaccg cattggccat caaactgatg ttgatgggtt ag 702
<210> 7
   <211> 233
   <212> PRT
   <213> Corynebacterium glutamicum ATCC13869
<220>
   <221> LysE
   <222> (1)..(233)
<400> 7
   Met Glu Ile Phe Ile Thr Gly Leu Leu Leu Gly Ala Ser Leu Leu Leu
   1 5 10 15
   Ser Ile Gly Pro Gln Asn Val Leu Val Ile Lys Gln Gly Ile Lys Arg
   20 25 30
   Glu Gly Leu Ile Ala Val Leu Leu Val Cys Leu Ile Ser Asp Val Phe
           35                  40                  45
   Leu Phe Ile Ala Gly Thr Leu Gly Val Asp Leu Leu Ser Asn Ala Ala
       50                  55                  60
   Pro Ile Val Leu Asp Ile Met Arg Trp Gly Gly Ile Ala Tyr Leu Leu
   65                  70                  75                  80
   Trp Phe Ala Val Met Ala Ala Lys Asp Ala Met Thr Asn Lys Val Glu
                   85                  90                  95
   Ala Pro Gln Ile Ile Glu Glu Thr Glu Pro Thr Val Pro Asp Asp Thr
               100                 105                 110
   Pro Leu Gly Gly Ser Ala Val Ala Thr Asp Thr Arg Asn Arg Val Arg
           115                 120                 125
   Val Glu Val Ser Val Asp Lys Gln Arg Val Trp Val Lys Pro Met Leu
       130                 135                 140
   Met Ala Ile Val Leu Thr Trp Leu Asn Pro Asn Ala Tyr Leu Asp Ala
   145                 150                 155                 160
   Phe Val Phe Ile Gly Gly Val Gly Ala Gln Tyr Gly Asp Thr Gly Arg
                   165                 170                 175
   Trp Ile Phe Ala Ala Gly Ala Phe Ala Ala Ser Leu Ile Trp Phe Pro
               180                 185                 190
   Leu Val Gly Phe Gly Ala Ala Ala Leu Ser Arg Pro Leu Ser Ser Pro
           195                 200                 205
   Lys Val Trp Arg Trp Ile Asn Val Val Val Ala Val Val Met Thr Ala
       210                 215                 220
   Leu Ala Ile Lys Leu Met Leu Met Gly
   225                 230
<210> 8
   <211> 702
   <212> DNA
   <213> Corynebacterium glutamicum ATCC13869
<220>
   <221> lysE-CDS
   <222> (1)..(702)
<400> 8
   atggaaatct tcattacagg tctgcttttg ggggccagtc ttttgctgtc catcggaccg      60
   cagaatgtac tggtgattaa acaaggaatt aagcgcgaag gactcattgc ggttcttctc     120
   gtgtgtttaa tttctgacgt ctttttgttc atcgccggca ccttgggcgt tgatcttttg     180
   tccaatgccg cgccgatcgt gctcgatatt atgcgctggg gtggcatcgc ttacctgtta     240
   tggtttgccg tcatggcagc gaaagacgcc atgacaaaca aggtggaagc gccacagatc     300
   attgaagaaa cagaaccaac cgtgcccgat gacacgcctt tgggcggttc ggcggtggcc     360
   actgacacgc gcaaccgggt gcgggtggag gtgagcgtcg ataagcagcg ggtttgggtg     420
   aagcccatgt tgatggcaat cgtgctgacc tggttgaacc cgaatgcgta tttggacgcg     480
   tttgtgttta tcggcggcgt cggcgcgcaa tacggcgaca ccggacggtg gattttcgcc     540
   gctggcgcgt tcgcggcaag cctgatctgg ttcccgctgg tgggtttcgg cgcagcagca     600
   ttgtcacgcc cgctgtccag ccccaaggtg tggcgctgga tcaacgtcgt cgtggcagtt     660
   gtgatgaccg cattggccat caaactgatg ttgatgggtt ag                        702
<210> 9
   <211> 228
   <212> PRT
   <213> Corynebacterium efficiens YS-314
<220>
   <221> LysE
   <222> (1)..(228)
<400> 9
   Met Glu Ile Phe Val Thr Gly Leu Leu Leu Gly Ala Ser Leu Leu Leu
   1               5                   10                  15
   Ala Ile Gly Pro Gln Asn Val Leu Val Ile Lys Gln Gly Ile Lys Arg
               20                  25                  30
   Glu Gly Ile Thr Ala Val Ile Ile Val Cys Leu Leu Ser Asp Val Val
           35                  40                  45
   Leu Phe Thr Leu Gly Thr Leu Gly Val Gly Leu Ile Ser Asp Thr Ala
       50                  55                  60
   Pro Ile Ile Leu Asp Ile Leu Arg Trp Cys Gly Ile Ala Tyr Leu Leu
   65                  70                  75                  80
   Trp Phe Ala Val Met Ala Ala Arg Asp Ala Leu Arg Ala Arg Thr Glu
                   85                  90                  95
   Val Thr Phe Val Glu His Ser Glu Pro Val Ala Ala Ala Ser Ala Ser
               100                 105                 110
   Gly Gly Gly Val Thr Thr Lys Gln Arg Pro Arg Leu Arg Ile Thr Ser
           115                 120                 125
   Gly Thr Arg Gln Val Trp Val Arg Pro Met Leu Met Ala Ile Val Leu
       130                 135                 140
   Thr Trp Leu Asn Pro Asn Ala Tyr Leu Asp Ala Phe Val Phe Ile Gly
   145                 150                 155                 160
   Gly Val Gly Ala Gln Tyr Gly Glu Thr Gly Arg Trp Ile Phe Ala Ala
                   165                 170                 175
   Gly Ala Phe Ala Ala Ser Leu Val Trp Phe Pro Leu Val Gly Tyr Gly
               180                 185                 190
   Ala Ala Ala Leu Ser Arg Pro Leu Ser Ser Pro Arg Val Trp Arg Trp
           195                 200                 205
   Ile Asn Ile Gly Val Ala Val Val Leu Thr Gly Leu Ala Val Lys Leu
       210                 215                 220
   Ile Leu Met Gly
   225
<210> 10
   <211> 228
   <212> PRT
   <213> Corynebacterium diphteriae NCTC13129
<220>
   <221> LysE
   <222> (1)..(228)
<400> 10
   Met Ser Ile Ala Ile Ala Gly Phe Leu Met Gly Leu Ser Leu Ile Val
   1               5                   10                  15
   Ala Ile Gly Pro Gln Asn Ala Leu Ile Ile Arg Gln Gly Ile Lys Arg
               20                  25                  30
   Glu Gly Leu Ile Pro Ile Leu Val Val Cys Ile Leu Ser Asp Val Ile
           35                  40                  45
   Leu Ile Phe Gly Gly Thr Ala Gly Val Gly Ala Leu Val Asp Arg Ala
       50                  55                  60
   Pro Ile Ala Leu Val Val Leu Lys Trp Leu Gly Val Ala Tyr Leu Leu
   65 70 75 80
   Tyr Phe Gly Phe Thr Cys Phe Lys Glu Ala Phe Lys Arg His Gly Gln
   85 90 95
   Ala Leu Ala Val Glu Gln Ser Glu Pro Val Ala Tyr Glu Pro Val Ala
   100 105 110
   Asp Ala Ser Ser Gly Val Ile Thr Lys Thr Arg Thr Lys Ala Gln Pro
   115 120 125
   Lys Ser Ala Gln Arg Thr Trp Val Lys Pro Val Leu Ala Ala Leu Ala
   130 135 140
   Phe Thr Trp Leu Asn Pro Ala Ala Tyr Ile Asp Val Leu Val Met Leu
   145 150 155 160
   Gly Gly Ile Ala Asn Gln His Gly Pro Asp Gly Arg Trp Val Phe Ala
   165 170 175
   Leu Gly Ala Leu Cys Ala Ser Leu Thr Trp Phe Pro Phe Ile Gly Tyr
   180 185 190
   Thr Ser Thr Arg Phe Ser Thr Val Leu Ser Arg Pro Ala Val Trp Arg
   195 200 205
   Tyr Ile Asn Ile Ala Ile Gly Ile Ile Met Met Ile Met Cys Ala Arg
   210 215 220
   Leu Ile Met His
   225
<210> 11
   <211> 222
   <212> PRT
   <213> Corynebacterium striatum ATCC6940
<220>
   <221> LysE
   <222> (1)..(222)
<400> 11
   Met Ser Val Leu Leu Ala Gly Phe Leu Leu Gly Leu Ser Leu Ile Val
   1 5 10 15
   Ala Ile Gly Pro Gln Asn Ala Tyr Ile Ile Lys Met Gly Val Lys Arg
   20 25 30
   Asp His Ile Gly Ala Ile Ile Leu Ala Cys Leu Leu Ser Asp Val Ile
   35 40 45
   Leu Ile Asn Ala Gly Val Gly Gly Met Gly Val Leu Val Glu Lys Phe
   50 55 60
   Pro Thr Gly Leu Ile Ile Met Lys Tyr Leu Gly Ala Ala Tyr Leu Ile
   65 70 75 80
   Tyr Phe Gly Phe Thr Cys Phe Arg Asp Ala Phe Lys Lys Glu Gln Glu
   85 90 95
   Ala Leu Val Val Ser Ser Thr Pro Pro Ser Ala Pro Asn Glu Thr Glu
   100 105 110
   Leu Gly Gly Ala Thr Thr Val Met Thr Lys Gln Arg Thr Lys Ser Arg
   115 120 125
   Thr Trp Val Lys Pro Val Met Gly Ala Met Ala Leu Thr Trp Leu Asn
   130 135 140
   Pro Leu Ala Tyr Val Asp Val Leu Val Met Leu Gly Gly Ile Ala Gln
   145 150 155 160
   His Tyr Gly Asp Gln Arg Trp Val Phe Ala Ala Gly Ala Ile Met Ala
   165 170 175
   Ser Ala Val Trp Phe Pro Thr Val Gly Tyr Gly Ala Phe Lys Leu Ser
   180 185 190
   His Val Leu Ala Lys Pro Thr Thr Trp Arg Tyr Val Asn Phe Ala Ile
   195 200 205
   Gly Cys Val Met Met Leu Leu Thr Ala Lys Leu Leu Leu His
   210 215 220
<210> 12
   <211> 235
   <212> PRT
   <213> Corynebacterium aurimucosum ATCC700975
<220>
   <221> LysE
   <222> (1)..(235)
<400> 12
   Met Arg Arg Leu Glu Ala Met Ser Val Leu Leu Ala Gly Phe Ala Leu
   1               5                   10                  15
   Gly Leu Ser Leu Ile Ile Ala Ile Gly Pro Gln Asn Ala Tyr Ile Ile
               20                  25                  30
   Lys Met Gly Ile Lys Arg Asp His Val Gly Pro Ile Leu Leu Ala Cys
           35                  40                  45
   Leu Leu Ser Asp Val Ile Leu Ile Thr Gly Gly Thr Ala Gly Val Gly
       50                  55                  60
   Val Leu Val Glu Arg Phe Pro Thr Ala Leu Val Val Val Lys Tyr Leu
   65                  70                  75                  80
   Gly Ala Ala Tyr Leu Ile Tyr Phe Gly Phe Thr Cys Phe Arg Asp Ala
                   85                  90                  95
   Phe Lys Lys Gln Gln Asp Ala Leu Val Ile Glu Glu Thr Thr Pro Val
               100                 105                 110
   Ala Gln Val Val Asp Glu Asn Ser Gly Asn Ala Gly Ala Pro Gly Thr
           115                 120                 125
   Ser Val Leu Thr Lys Ile Arg Pro Arg Val Arg Ser Lys Ser Trp Val
       130                 135                 140
   Lys Pro Val Leu Gly Ala Leu Ala Leu Thr Trp Leu Asn Pro Leu Ala
   145                 150                 155                 160
   Tyr Val Asp Ala Leu Val Met Leu Gly Ser Ile Ala Asn Gln Tyr Gly
                   165                 170                 175
   Asp Gln Arg Trp Val Phe Ala Gly Gly Ala Ile Leu Ala Ser Ala Val
               180                 185                 190
   Trp Phe Pro Ser Leu Gly Phe Gly Ala Tyr Lys Leu Ser His Val Leu
           195                 200                 205
   Ala Lys Pro Thr Thr Trp Arg Val Val Asn Ile Val Ile Gly Cys Val
       210                 215                 220
   Met Leu Ala Leu Thr Ala Lys Leu Leu Phe Leu
   225                 230                 235
<210> 13
   <211> 244
   <212> PRT
   <213> Corynebacterium matruchotii ATCC33806
<220>
   <221> LysE
   <222> (1)..(244)
<400> 13
   Met Ser Ile Ala Val Ala Gly Phe Leu Leu Gly Leu Ser Leu Ile Val
   1               5                   10                  15
   Ala Ile Gly Pro Gln Asn Ala Leu Val Ile Arg Gln Gly Val Lys Arg
               20                  25                  30
   Glu Gly Leu Ile Val Val Leu Ala Ile Cys Ile Leu Ser Asp Ile Phe
           35                  40                  45
   Leu Ile Phe Gly Gly Thr Ala Gly Val Gly Val Ile Ile Glu Lys Ala
       50                  55                  60
   Pro Leu Ala Leu Val Ala Leu Lys Trp Phe Gly Ala Ala Tyr Leu Ala
   65                  70                  75                  80
   Trp Phe Ala Val Ser Cys Phe Arg Asp Met Val Lys Pro Arg Ala Leu
                   85                  90                  95
   Asp Ser Ser Ala Thr Asp Asp Gly Thr Ser Leu Asp Asp Ala Pro Thr
               100                 105                 110
   Ala Ala His Val Ser Asn Val Asp Thr Thr Ser Gly Asn Gly Gly Gln
           115                 120                 125
   Val Gln Thr Lys Thr Arg Pro Ile Thr Thr Thr Ala Pro Thr Arg Gln
       130                 135                 140
   Ala His Pro Ala Arg Pro Trp Val Lys Pro Ala Leu Ala Ala Leu Ala
   145                 150                 155                 160
   Phe Thr Trp Leu Asn Pro Ser Ala Tyr Ile Asp Thr Leu Val Met Leu
                   165                 170                 175
   Gly Gly Ile Ala Asn Gln His Gly Glu Ser Gly Arg Trp Val Phe Ala
               180                 185                 190
   Ala Gly Ala Leu Met Ala Ser Ala Val Trp Phe Pro Leu Leu Gly Phe
           195                 200                 205
   Phe Ser Thr Arg Phe Ser Arg Val Leu Ser Arg Pro Gln Ala Trp Arg
       210                 215                 220
   Val Ile Asn Gly Val Ile Gly Cys Ile Met Val Val Met Cys Ile Arg
   225                 230                 235                 240
   Leu Val Met His
<210> 14
   <211> 230
   <212> PRT
   <213> Corynebacterium pseudogenitalium ATCC33035
<220>
   <221> LysE
   <222> (1)..(230)
<400> 14
   Met Ser Ile Val Leu Ala Gly Phe Phe Leu Gly Leu Ser Leu Ile Val
   1               5                   10                  15
   Ala Val Gly Pro Gln Asn Ala Met Leu Leu Lys Tyr Gly Ile Arg Arg
               20                  25                  30
   Asp His Ile Gly Leu Ile Ile Val Val Cys Ala Leu Ser Asp Val Ile
           35                  40                  45
   Leu Ile Thr Ser Gly Thr Ala Gly Val Gly Tyr Leu Val Glu Lys Phe
       50                  55                  60
   Pro Asn Ala Leu Gln Val Leu Lys Tyr Val Gly Ala Ala Tyr Leu Ala
   65                  70                  75                  80
   Tyr Phe Thr Phe Thr Cys Phe Arg Asp Ala Phe Lys Thr Lys Gly Glu
                   85                  90                  95
   Ala Ile Glu Val Glu Ser Thr Gln Pro Lys Ala Pro Gln Glu Val Ala
               100                 105                 110
   Ser Phe Asp Gly Ser Gln Ala Arg Ser Thr Thr Lys Thr Ala Ala Arg
           115                 120                 125
   Val Glu Ile Lys Arg Ser Pro Ser Trp Val Lys Pro Leu Leu Thr Ala
       130                 135                 140
   Leu Ala Leu Thr Trp Leu Asn Pro Gly Ala Tyr Val Asp Val Val Val
   145                 150                 155                 160
   Met Leu Gly Ser Ile Ala Asn Gln Tyr Gly Glu Ser Gly Arg Trp Leu
                   165                 170                 175
   Phe Ala Val Gly Ala Ile Cys Ala Ser Phe Thr Trp Phe Pro Phe Ile
               180                 185                 190
   Gly Phe Gly Ala Ala Arg Phe Ser His Val Leu Ser Arg Pro Thr Val
           195                 200                 205
   Trp Arg Trp Ile Asn Phe Gly Ile Gly Val Ile Met Ile Gly Leu Thr
       210                 215                 220
   Leu Lys Leu Leu Leu Leu
   225                 230
<210> 15
   <211> 241
   <212> PRT
   <213> Corynebacterium accolens ATCC49725
<220>
   <221> LysE
   <222> (1)..(241)
<400> 15
   Met Ser Ile Val Leu Ala Gly Phe Val Leu Gly Leu Ser Leu Ile Val
   1               5                   10                  15
   Ala Val Gly Pro Gln Asn Ala Met Leu Leu Lys Tyr Gly Ile Arg Arg
               20                  25                  30
   Asp His Ile Gly Leu Ile Ile Val Val Cys Ala Leu Ser Asp Val Ile
           35                  40                  45
   Leu Ile Thr Ser Gly Thr Ala Gly Val Gly Tyr Leu Val Glu Arg Phe
       50                  55                  60
   Pro Asn Ala Leu Glu Ala Leu Lys Tyr Ile Gly Ala Ala Tyr Leu Ala
   65                  70                  75                  80
   Phe Phe Thr Phe Thr Cys Phe Arg Asp Ala Phe Lys Thr Lys Gly Glu
                   85                  90                  95
   Ala Ile Asp Val Glu Ser Thr Ser Pro Asn Ser Thr Glu Glu Val Ala
               100                 105                 110
   Thr Phe Asp Gly Asp Gly Asp Ser Thr Gly Gly Val Gly Thr Glu His
           115                 120                 125
   Gly Ser Val Ala Thr Ala Thr Ala Thr Gln Arg Gln Glu Ile Lys Arg
       130                 135                 140
   Ser Pro Ser Trp Val Lys Pro Leu Leu Thr Ala Leu Ala Leu Thr Trp
   145                 150                 155                 160
   Leu Asn Pro Gly Ala Tyr Val Asp Val Leu Val Met Leu Gly Gly Ile
                   165                 170                 175
   Ala Asn Gln Tyr Gly Asp Pro Gly Arg Trp Leu Phe Ala Gly Gly Ala
               180                 185                 190
   Ile Ala Ala Ser Phe Thr Trp Phe Pro Val Ile Gly Phe Gly Ala Ala
           195                 200                 205
   Arg Phe Ser His Val Leu Ser Arg Pro Glu Val Trp Arg Trp Ile Asn
       210                 215                 220
   Val Gly Ile Gly Val Ile Met Ile Gly Leu Thr Leu Lys Leu Leu Leu
   225                 230                 235                 240
   Leu
<210> 16
   <211> 261
   <212> PRT
   <213> Corynebacterium glucuronalyticum ATCC51867
<400> 16
   Met Val Pro Glu Asn Leu Ser Phe Tyr Leu Cys Val Leu Leu Thr His
   1               5                   10                  15
   Asn Lys Tyr Val Asn Val Phe Phe Ala Gly Leu Leu Phe Asn Leu Ser
               20                  25                  30
   Leu Ile Leu Ala Leu Gly Pro Gln Asn Ala Leu Ile Leu Lys Tyr Gly
           35                  40                  45
   Leu Arg Arg Gln Ala Ile Thr Leu Val Ile Ser Val Cys Ala Leu Cys
       50                  55                  60
   Asp Ile Thr Leu Ile Ala Leu Ser Gly Val Gly Val Gly Val Ile Leu
   65                  70                  75                  80
   Gln Lys Ala Pro Ile Val Leu Glu Ile Leu Arg Tyr Ala Gly Phe Leu
                   85                  90                  95
   Tyr Leu Leu Trp Phe Ala Tyr Thr Cys Phe Arg Asp Ala Ile His Pro
   100 105 110
   Lys Thr Leu Ala Thr Glu Thr Val Ser Glu Thr Lys Pro His Glu Glu
   115 120 125
   Glu Leu Pro Asp Val Ser Ser Thr Thr Ala Gly Thr Thr Met Ala Thr
   130 135 140
   Ala Thr Val Met Glu Thr Ala Thr Thr Val Lys Glu Lys Thr His Arg
   145 150 155 160
   Arg Thr Phe His Ile Pro Gln Glu Ile Lys Gly Pro Ala Val Ala Ala
   165 170 175
   Phe Val Val Ser Val Ile Asn Pro Ala Ala Trp Val Asp Leu Phe Val
   180 185 190
   Val Ile Gly Ser Ile Ser Ser Ser Tyr Gly Pro Asp Lys Trp Ala Phe
   195 200 205
   Leu Leu Gly Thr Met Ala Ala Ser Leu Val Trp Phe Pro Ala Phe Gly
   210 215 220
   Tyr Gly Ala Ala Ala Leu Ser Arg Pro Leu Ser Ser Pro Lys Val Trp
   225 230 235 240
   Arg Cys Ile Asn Thr Gly Ile Gly Leu Phe Met Val Phe Met Ala Phe
   245 250 255
   Arg Val Leu Phe Met
   260
<210> 17
   <211> 204
   <212> PRT
   <213> Micrococcus luteus NCTC2665
<400> 17
   Met Trp Thr Leu Ala Gly Thr Gly Leu Leu Thr Gly Leu Ala Leu Ile
   1               5                   10                  15
   Val Ala Ile Gly Ala Gln Asn Ala Phe Val Leu Arg Gln Gly Val Arg
               20                  25                  30
   Arg Glu His Val Gly Ala Val Val Leu Val Cys Met Ala Ser Asp Ala
           35                  40                  45
   Val Leu Ile Leu Ala Gly Thr Ala Gly Val Gly Ala Leu Val Gln Ala
       50                  55                  60
   Val Pro Trp Leu Leu Glu Val Leu Arg Trp Gly Gly Ala Leu Tyr Leu
   65                  70                  75                  80
   Leu Trp Phe Ala Val Ser Ser Leu Arg Ala Ala Leu Arg Pro Gln Gly
                   85                  90                  95
   Leu Met Ala Glu Gln Ala Pro Arg Thr Ala Gly Ser Val Ile Ala Thr
               100                 105                 110
   Thr Leu Ala Leu Thr Trp Leu Asn Pro His Val Tyr Leu Asp Thr Val
           115                 120                 125
   Val Leu Leu Gly Ser Leu Ala Asn Gln His Gly Pro Asp Ala Arg Trp
       130                 135                 140
   Val Phe Ala Ala Gly Ala Val Ala Ala Ser Val Leu Trp Phe Thr Ala
   145                 150                 155                 160
   Leu Gly Tyr Gly Ala Arg Leu Leu Ala Arg Val Leu Ala Asp Pro Lys
                   165                 170                 175
   Ala Trp Arg Val Val Asp Val Val Ile Ala Val Val Met Ala Val Leu
               180                 185                 190
   Ala Val Arg Leu Ile Ala Gly Ser Asp Val Trp Gly
           195                 200
<210> 18
   <211> 230
   <212> PRT
   <213> Corynebacterium tubuculostearicum SK141
<220>
   <221> ArgO(LysE)
   <222> (1)..(230)
<400> 18
   Met Ser Ile Val Leu Ala Gly Phe Phe Leu Gly Leu Ser Leu Ile Val
   1               5                   10                  15
   Ala Val Gly Pro Gln Asn Ala Met Leu Leu Lys Tyr Gly Ile Arg Arg
               20                  25                  30
   Asp His Ile Gly Leu Ile Ile Val Val Cys Ala Leu Ser Asp Val Ile
           35                  40                  45
   Leu Ile Thr Ser Gly Thr Ala Gly Val Gly Tyr Leu Val Glu Lys Phe
       50                  55                  60
   Pro Asn Ala Leu Gln Val Leu Lys Tyr Val Gly Ala Ala Tyr Leu Ala
   65                  70                  75                  80
   Tyr Phe Thr Phe Thr Cys Phe Arg Asp Ala Leu Lys Thr Lys Gly Glu
                   85                  90                  95
   Ala Ile Glu Val Glu Ser Thr Gln Pro Lys Val Pro Gln Glu Val Ala
               100                 105                 110
   Ser Phe Asp Gly Ser Gln Ala Arg Asn Thr Thr Lys Thr Ala Thr Arg
           115                 120                 125
   Val Glu Ile Lys Arg Ser Pro Ser Trp Val Lys Pro Leu Leu Thr Ala
       130                 135                 140
   Leu Ala Leu Thr Trp Leu Asn Pro Gly Ala Tyr Val Asp Val Val Val
   145                 150                 155                 160
   Met Leu Gly Ser Ile Ala Asn Gln Tyr Gly Glu Ser Gly Arg Trp Leu
                   165                 170                 175
   Phe Ala Val Gly Ala Ile Cys Ala Ser Phe Thr Trp Phe Pro Phe Ile
               180                 185                 190
   Gly Phe Gly Ala Ala Arg Phe Ser His Val Leu Ser Arg Pro Thr Val
           195                 200                 205
   Trp Arg Trp Ile Asn Phe Gly Ile Gly Val Ile Met Ile Gly Leu Thr
       210                 215                 220
   Leu Lys Leu Leu Leu Leu
   225                 230
<210> 19
   <211> 244
   <212> PRT
   <213> Corynebacterium matruchotii ATCC14266
<220>
   <221> ArgO(LysE)
   <222> (1)..(244)
<400> 19
   Met Ser Ile Ala Val Ala Gly Phe Leu Leu Gly Leu Ser Leu Ile Val
   1               5                   10                  15
   Ala Ile Gly Pro Gln Asn Ala Leu Val Ile Arg Gln Gly Val Lys Arg
               20                  25                  30
   Glu Gly Leu Ile Val Val Leu Ala Ile Cys Met Leu Ser Asp Ile Phe
           35                  40                  45
   Leu Ile Phe Gly Gly Thr Ala Gly Val Gly Val Ile Ile Glu Lys Ala
       50                  55                  60
   Pro Leu Ala Leu Val Ala Leu Lys Trp Phe Gly Ala Ala Tyr Leu Ala
   65                  70                  75                  80
   Trp Phe Ala Val Ser Cys Phe Lys Asp Met Val Lys Pro Arg Ala Leu
                   85                  90                  95
   Asp Ser Ser Ala Thr Asp Asn Gly Thr Ser Leu Asp Asp Ala Pro Thr
               100                 105                 110
   Val Ala His Ile Ser Asn Val Asp Ser Thr Ser Gly Asn Gly Gly Gln
           115                 120                 125
   Val Gln Thr Lys Thr Arg Pro Ile Thr Thr Thr Ala Pro Thr Arg Gln
       130                 135                 140
   Ala His Pro Ala Arg Pro Trp Val Lys Pro Ala Leu Ala Ala Leu Ala
   145                 150                 155                 160
   Phe Thr Trp Leu Asn Pro Ser Ala Tyr Ile Asp Thr Leu Val Met Leu
                   165                 170                 175
   Gly Gly Ile Ala Asn Gln His Gly Glu Ser Gly Arg Trp Val Phe Ala
               180                 185                 190
   Ala Gly Ala Leu Met Ala Ser Ala Val Trp Phe Pro Leu Leu Gly Phe
           195                 200                 205
   Phe Ser Thr Arg Phe Ser Arg Val Leu Ser Arg Pro Gln Ala Trp Arg
       210                 215                 220
   Val Ile Asn Gly Val Ile Gly Cys Ile Met Val Val Met Cys Ile Arg
   225                 230                 235                 240
   Leu Ile Met His
<210> 20
   <211> 864
   <212> DNA
   <213> Corynebacterium glutamicum ATCC13032
<220>
   <221> lysE
   <222> (76)..(777)
   <223> pVWEx1- Insert
<400> 20
   tgagcggata acaatttcac acaggaaaca gaattaaaag atatgaccat gattacgcca      60
   agcttgcatg ccatcatgga aatcttcatt acaggtctgc ttttgggggc cagtctttta     120
   ctgtccatcg gaccgcagaa tgtactggtg attaaacaag gaattaagcg cgaaggactc     180
   attgcggttc ttctcgtgtg tttaatttct gacgtctttt tgttcatcgc cggcaccttg     240
   ggcgttgatc ttttgtccaa tgccgcgccg atcgtgctcg atattatgcg ctggggtggc     300
   atcgcttacc tgttatggtt tgccgtcatg gcagcgaaag acgccatgac aaacaaggtg     360
   gaagcgccac agatcattga agaaacagaa ccaaccgtgc ccgatgacac gcctttgggc     420
   ggttcggcgg tggccactga cacgcgcaac cgggtgcggg tggaggtgag cgtcgataag     480
   cagcgggttt gggtaaagcc catgttgatg gcaatcgtgc tgacctggtt gaacccgaat     540
   gcgtatttgg acgcgtttgt gtttatcggc ggcgtcggcg cgcaatacgg cgacaccgga     600
   cggtggattt tcgccgctgg cgcgttcgcg gcaagcctga tctggttccc gctggtgggt     660
   ttcggcgcag cagcattgtc acgcccgctg tccagcccca aggtgtggcg ctggatcaac     720
   gtcgtcgtgg cagttgtgat gaccgcattg gccatcaaac tgatgttgat gggttagttt     780
   tcgcgggttt tggaatcggt ggccttcgcc caaatattga cctagaggat ccccgggtac     840
   cgagctcgaa ttcactggcc gtcg                                            864
<210> 21
   <211> 1036
   <212> DNA
   <213> Corynebacterium glutamicum ATCC13032
<220>
   <221> 'argD
   <222> (15)..(491)
   <223> 3'-Ende des argD-Gens
<220>
   <221> argF'
   <222> (505)..(523)
   <223> 5'-Ende des argF-Gens
<220>
   <221> 'argH
   <222> (524)..(538)
   <223> 3'-Ende des argH-Gens
<220>
   <221> cg1589'
   <222> (605)..(1024)
   <223> 5'-Ende des cg1589-Leserasters
<400> 21
   ggtggtgcta gcccggcgat ttctttgcac atcagcacga tggcgttgtt cccgatgtgg      60
   tgaccatggc caagggactt ggcggcggtc ttcccatcgg tgcttgtttg gccactggcc     120
   gtgcagctga attgatgacc ccaggcaagc acggcaccac tttcggtggc aacccagttg     180
   cttgtgcagc tgccaaggca gtgctgtctg ttgtcgatga cgctttctgc gcagaagttg     240
   cccgcaaggg cgagctgttc aaggaacttc ttgccaaggt tgacggcgtt gtagacgtcc     300
   gtggcagggg cttgatgttg ggcgtggtgc tggagcgcga cgtcgcaaag caagctgttc     360
   ttgatggttt taagcacggc gttattttga atgcaccggc ggacaacatt atccgtttga     420
   ccccgccgct ggtgatcacc gacgaagaaa tcgcagacgc agtcaaggct attgccgaga     480
   caatcgcata aaggactcaa acttatgact tcacaaccac aggggggtac gtcgataagc     540
   attagtttat ggcctgtgct gctttccgat tgcgggagtt gcacaggcca tttattatca     600
   attcatgaat ggttcccctg attactcaag aaaatctcga ggcaggggat tttccgtatt     660
   tttaggcatc atcctgctgg tcatcgcggt attagcggtc ttggtgggcc gcggaaccat     720
   cgccatgcca aagctcttcg gatcaagtaa cttgaccgag gtcagagcag taattggctc     780
   ggaaaagaag gaattcttcg aagatccaga agtcgttgaa gccttcgccg accacggctt     840
   tgaagttaac gtggacaccg caggatctcg tcggatcgcc actgatgtgg acttgagccc     900
   gtacgatttc gcgttcccat cctctgcacc agctgcgcag aagatctccg aagccaacac     960
   cacgacgggc cgattcaccc cattctattc gcctatggcg gtagcaacat tcggaaccat    1020
   caccatgcat accacc                                                    1036
<210> 22
   <211> 28
   <212> DNA
   <213> Oligonukleotid
<220>
   <221> lysE_1.p
   <222> (1)..(28)
<400> 22
   tcgatatcat ggaaatcttc attacagg 28
<210> 23
   <211> 32
   <212> DNA
   <213> Oligonukleotid
<220>
   <221> lysE_2.p
   <222> (1)..(32)
<400> 23
   tgcctaggtc aatatttggg cgaaggccac cg 32
<210> 24
   <211> 32
   <212> DNA
   <213> Oligonukleotid
<220>
   <221> argFRGH_d1
   <222> (1)..(32)
<400> 24
   ggtggtgcta gcccggcgat ttctttgcac at 32
<210> 25
   <211> 40
   <212> DNA
   <213> Oligonukleotid
<220>
   <221> argFRGH_d2
   <222> (1)..(40)
<400> 25
   aatgcttatc gacgtacccc cctgtggttg tgaagtcata 40
<210> 26
   <211> 20
   <212> DNA
   <213> Oligonukleotid
<220>
   <221> argFRGH_d3
   <222> (1)..(20)
<400> 26
   ggggtacgtc gataagcatt 20
<210> 27
   <211> 32
   <212> DNA
   <213> Oligonukleotid
<220>
   <221> argFRGH_d4
   <222> (1)..(32)
<400> 27
   ggtggtatgc atggtgatgg ttccgaatgt tg 32
<210> 28
   <211> 238
   <212> DNA
   <213> PgapRBS Corynebacterium glutamicum ATCC13032
<220>
   <221> RBS
   <222> (225)..(232)
<400> 28
   ctgtatgatt ttgcatctgc tgcgaaatct ttgtttcccc gctaaagttg aggacaggtt     60
   gacacggagt tgactcgacg aattatccaa tgtgagtagg tttggtgcgt gagttggaaa    120
   aattcgccat actcgccctt gggttctgtc agctcaagaa ttcttgagtg accgatgctc    180
   tgattgacct aactgcttga cacattgcat ttcctacaat ctttagagga gacacaac      238
<210> 29
   <211> 8281
   <212> DNA
   <213> pEC71ysE
<400> 29
   tcccagaggc agcgtcggcg gctcctgcct cccccgcccg gcgccgaccg ggacccgcaa     60
   accccttgat ccgctgtcgg gggtgatcct gcaagcctcg tcgtcctggc cggaccacgc    120
   tatctgtgca aggtccccgg ccccggacgc gcgctccatg agcagagcgc ccgccgggga    180
   tcgatccggg cttatcgact gcacggtgca ccaatgcttc tggcgtcagg cagccatcgg    240
   aagctgtggt atggctgtgc aggtcgtaaa tcactgcata attcgtgtcg ctcaaggcgc    300
   actcccgttc tggataatgt tttttgcgcc gacatcataa cggttctggc aaatattctg    360
   aaatgagctg ttgacaatta atcatcggct cgtataatgt gtggaattgt gagcggataa    420
   caatttcaca caggaaacag aattcccagc ttgagtagga caaatccgcc gagcttcgac    480
   gagattttca ggagctaagg aagctaaaat ggagaaaaaa atcactggat ataccaccgt    540
   tgatatatcc caatggcatc gtaaagaaca ttttgaggca tttcagtcag ttgctcaatg    600
   tacctataac cagaccgttc agctggatat tacggccttt ttaaagaccg taaagaaaaa    660
   taagcacaag ttttatccgg cctttattca cattcttgcc cgcctgatga atgctcatcc    720
   ggaattccgt atggcaatga aagacggtga gctggtgata tgggatagtg ttcacccttg    780
   ttacaccgtt ttccatgagc aaactgaaac gttttcatcg ctctggagtg aataccacga    840
   cgatttccgg cagtttctac acatatattc gcaagatgtg gcgtgttacg gtgaaaacct    900
   ggcctatttc cctaaagggt ttattgagaa tatgtttttc gtctcagcca atccctgggt    960
   gagtttcacc agttttgatt taaacgtggc caatatggac aacttcttcg cccccgtttt   1020
   caccatgggc aaatattata cgcaaggcga caaggtgctg atgccgctgg cgattcaggt   1080
   tcatcatgcc gtttgtgatg gcttccatgt cggcagaatg cttaatgaat tacaacagta   1140
   ctgcgatgag tggcagggcg gggcgtaatt tttttaaggc agttattggt gcccttaaac 1200
   gcctggttgc tacgcctgaa taagtgataa taagcggatg aatggcagaa attcgtcgag 1260
   gcggcacctc gctaacggat tcaccactcc aagaattgga gccaatcaat tcttgcggag 1320
   aactgtgaat gcgcaaacca acccttggca gaacatatcc atcgcgtccg ccatctccag 1380
   cagccgcacg cggcgcatct cggctgtttt ggcggatgag agaagatttt cagcctgata 1440
   cagattaaat cagaacgcag aagcggtctg ataaaacaga atttgcctgg cggcagtagc 1500
   gcggtggtcc cacctgaccc catgccgaac tcagaagtga aacgccgtag cgccgatggt 1560
   agtgtggggt ctccccatgc gagagtaggg aactgccagg catcaaataa aacgaaaggc 1620
   tcagtcgaaa gactgggcct ttcgttttat ctgttgtttg tcggtgaacg ctctcctgag 1680
   taggacaaat ccgccgggag cggatttgaa cgttgcgaag caacggcccg gagggtggcg 1740
   ggcaggacgc ccgccataaa ctgccaggca tcaaattaag cagaaggcca tcctgacgga 1800
   tggccttttt gcgtttctac aaactcttcc tgtcgtcata tctacaagcc atccccccac 1860
   agatacggta aactagcctc gtttttgcat caggaaagca gaattcgtaa tcatgtcata 1920
   gctgtttcct gtgtgaaatt gttatccgct cacaattcca cacaacatac gagccggaag 1980
   cataaagtgt aaagcctggg gtgcctaatg agtgagctaa ctcacattaa ttgcgttgcg 2040
   ctcactgccc gctttccagt cgggaaacct gtcgtgccag ctgcattaat gaatcggcca 2100
   acgcgcgggg agaggcggtt tgcgtattgg gcgctcttcc gcttcctcgc tcactgactc 2160
   gctgcgctcg gtcgttcggc tgcggcgagc ggtatcagct cactcaaagg cggtaatacg 2220
   gttatccaca gaatcagggg ataacgcagg aaagaacatg tgagcaaaag gccagcaaaa 2280
   ggccaggaac cgtaaaaagg ccgcgttgct ggcgtttttc cataggctcc gcccccctga 2340
   cgagcatcac aaaaatcgac gctcaagtca gaggtggcga aacccgacag gactataaag 2400
   ataccaggcg tttccccctg gaagctccct cgtgcgctct cctgttccga ccctgccgct 2460
   taccggatac ctgtccgcct ttctcccttc gggaagcgtg gcgctttctc atagctcacg 2520
   ctgtaggtat ctcagttcgg tgtaggtcgt tcgctccaag ctgggctgtg tgcacgaacc 2580
   ccccgttcag cccgaccgct gcgccttatc cggtaactat cgtcttgagt ccaacccggt 2640
   aagacacgac ttatcgccac tggcagcagc cactggtaac aggattagca gagcgaggta 2700
   tgtaggcggt gctacagagt tcttgaagtg gtggcctaac tacggctaca ctagaagaac 2760
   agtatttggt atctgcgctc tgctgaagcc agttaccttc ggaaaaagag ttggtagctc 2820
   ttgatccggc aaacaaacca ccgctggtag cggtggtttt tttgtttgca agcagcagat 2880
   tacgcgcaga aaaaaaggat ctcaagaaga tcctttgatc ttttctacgg ggtctgacgc 2940
   tcagtggaac gaaaactcac gttaagggat tttggtcatg agattatcaa aaaggatctt 3000
   cacctagatc cttttggggg gggggggaaa gccacgttgt gtctcaaaat ctctgatgtt 3060
   acattgcaca agataaaaat atatcatcat gaacaataaa actgtctgct tacataaaca 3120
   gtaatacaag gggtgttatg agccatattc aacgggaaac gtcttgctcg aagcccgcct 3180
   aatgagcggg ctttttttta aaaaaaagcc cgctcattag gcgggctagc ccgcctaatg 3240
   agcgggcttt ttttttcgag gccgcgatta aattccaaca tggatgctga tttatatggg 3300
   tataaatggg ctcgaaaaga tatgaccatg attacgccaa gcttgcatgc ctgcaggtcg 3360
   actctagagg atccaccgtg accagtgcat tgattggtgc ttcgtcagtt gagcagctgg 3420
   acaacagcct tgattcactc aacaacttgg agttttctga cgccgagttg gaggcgatcg 3480
   atgagatttc ccacgacgcc ggcatcaaca tttgggcgaa ggccaccgat tccaaaaccc 3540
   gcgaaaacta acccatcaac atcagtttga tggccaatgc ggtcatcaca actgccacga 3600
   cgacgttgat ccagcgccac accttggggc tggacagcgg gcgtgacaat gctgctgcgc 3660
   cgaaacccac cagcgggaac cagatcaggc ttgccgcgaa cgcgccagcg gcgaaaatcc 3720
   accgtccggt gtcgccgtat tgcgcgccga cgccgccgat aaacacaaac gcgtccaaat 3780
   acgcattcgg gttcaaccag gtcagcacga ttgccatcaa catgggcttt acccaaaccc 3840
   gctgcttatc gacgctcacc tccacccgca cccggttgcg cgtgtcagtg gccaccgccg 3900
   aaccgcccaa aggcgtgtca tcgggcacgg ttggttctgt ttcttcaatg atctgtggcg 3960
   cttccacctt gtttgtcatg gcgtctttcg ctgccatgac ggcaaaccat aacaggtaag 4020
   cgatgccacc ccagcgcata atatcgagca cgatcggcgc ggcattggac aaaagatcaa 4080
   cgcccaaggt gccggcgatg aacaaaaaga cgtcagaaat taaacacacg agaagaaccg 4140
   caatgagtcc ttcgcgctta attccttgtt taatcaccag tacattctgc ggtccgatgg 4200
   acagtaaaag actggccccc aaaagcagac ctgtaatgaa gatttccatg atcaccatcg 4260
   tgacctatgg aagtacttaa gtaaaatgat tggttcttaa catggtttaa tatagcttca 4320
   tgaaccccat tcaactggac actttgctct caatcattga tgaaggcagc ttcgaaggcg 4380
   cctccttagc cctttccatt tccccctcgg cggtgagtca gcgcggatcc ccgggtaccg 4440
   agctcgaatt cactggccgt cgttttaccg ataatgtcgg gcaatcaggt gcgacaatct 4500
   atcgagcttt tgccattctc accggattca gtcgtcactc atggtgattt ctcacttgat 4560
   aaccttattt ttgacgaggg gaaattaata ggttgtattg atgttggacg agtcggaatc 4620
   gcagaccgat accaggatct tgccatccta tggaactgcc tcggtgagtt ttctccttca 4680
   ttacagaaac ggctttttca aaaatatggt attgataatc ctgatatgaa taaattgcag 4740
   tttcatttga tgctcgatga gtttttctaa tcagaattgg ttaattggtt gtaacactgg 4800
   cagagcatta cgctgacttg acgggacggc ggctttgttg aataaatcga acttttgctg 4860
   agttgaagga tcagatcacg catcttcccg acaacgcaga ccgttccgtg gcaaagcaaa 4920
   agttcaaaat caccaactgg tccacctaca acaaagctct catcaaccgt ggctccctca 4980
   ctttctggct ggatgatggg gcgattcagg cctggtatga gtcagcaaca ccttcttcac 5040
   gaggcagacc tcagcgcccc ccccccccta gcttgtctac gtctgatgct ttgaatcgga 5100
   cggacttgcc gatcttgtat gcggtgattt ttccctcgtt tgcccacttt ttaatggtgg 5160
   ccggggtgag agctacgcgg gcggcgacct gctgcgctgt gatccaatat tcggggtcgt 5220
   tcactggttc ccctttctga tttctggcat agaagaaccc ccgtgaactg tgtggttccg 5280
   ggggttgctg atttttgcga gacttctcgc gcaattccct agcttaggtg aaaacaccat 5340
   gaaacactag ggaaacaccc atgaaacacc cattagggca gtagggcggc ttcttcgtct 5400
   agggcttgca tttgggcggt gatctggtct ttagcgtgtg aaagtgtgtc gtaggtggcg 5460
   tgctcaatgc actcgaacgt cacgtcattt accgggtcac ggtgggcaaa gagaactagt 5520
   gggttagaca ttgttttcct cgttgtcggt ggtggtgagc ttttctagcc gctcggtaaa 5580
   cgcggcgatc atgaactctt ggaggttttc accgttctgc atgcctgcgc gcttcatgtc 5640
   ctcacgtagt gccaaaggaa cgcgtgcggt gaccacgacg ggcttagcct ttgcctgcgc 5700
   ttctagtgct tcgatggtgg cttgtgcctg cgcttgctgc gcctgtagtg cctgttgagc 5760
   ttcttgtagt tgctgttcta gctgtgcctt ggttgccatg ctttaagact ctagtagctt 5820
   tcctgcgata tgtcatgcgc atgcgtagca aacattgtcc tgcaactcat tcattatgtg 5880
   cagtgctcct gttactagtc gtacatactc atatttacct agtctgcatg cagtgcatgc 5940
   acatgcagtc atgtcgtgct aatgtgtaaa acatgtacat gcagattgct gggggtgcag 6000
   ggggcggagc caccctgtcc atgcggggtg tggggcttgc cccgccggta cagacagtga 6060
   gcaccggggc acctagtcgc ggataccccc cctaggtatc ggacacgtaa ccctcccatg 6120
   tcgatgcaaa tctttaacat tgagtacggg taagctggca cgcatagcca agctaggcgg 6180
   ccaccaaaca ccactaaaaa ttaatagttc ctagacaaga caaacccccg tgcgagctac 6240
   caactcatat gcacgggggc cacataaccc gaaggggttt caattgacaa ccatagcact 6300
   agctaagaca acgggcacaa cacccgcaca aactcgcact gcgcaacccc gcacaacatc 6360
   gggtctaggt aacactgaaa tagaagtgaa cacctctaag gaaccgcagg tcaatgaggg 6420
   ttctaaggtc actcgcgcta gggcgtggcg taggcaaaac gtcatgtaca agatcaccaa 6480
   tagtaaggct ctggcggggt gccataggtg gcgcagggac gaagctgttg cggtgtcctg 6540
   gtcgtctaac ggtgcttcgc agtttgaggg tctgcaaaac tctcactctc gctgggggtc 6600
   acctctggct gaattggaag tcatgggcga acgccgcatt gagctggcta ttgctactaa 6660
   gaatcacttg gcggcgggtg gcgcgctcat gatgtttgtg ggcactgttc gacacaaccg 6720
   ctcacagtca tttgcgcagg ttgaagcggg tattaagact gcgtactctt cgatggtgaa 6780
   aacatctcag tggaagaaag aacgtgcacg gtacggggtg gagcacacct atagtgacta 6840
   tgaggtcaca gactcttggg cgaacggttg gcacttgcac cgcaacatgc tgttgttctt 6900
   ggatcgtcca ctgtctgacg atgaactcaa ggcgtttgag gattccatgt tttcccgctg 6960
   gtctgctggt gtggttaagg ccggtatgga cgcgccactg cgtgagcacg gggtcaaact 7020
   tgatcaggtg tctacctggg gtggagacgc tgcgaaaatg gcaacctacc tcgctaaggg 7080
   catgtctcag gaactgactg gctccgctac taaaaccgcg tctaaggggt cgtacacgcc 7140
   gtttcagatg ttggatatgt tggccgatca aagcgacgcc ggcgaggata tggacgctgt 7200
   tttggtggct cggtggcgtg agtatgaggt tggttctaaa aacctgcgtt cgtcctggtc 7260
   acgtggggct aagcgtgctt tgggcattga ttacatagac gctgatgtac gtcgtgaaat 7320
   ggaagaagaa ctgtacaagc tcgccggtct ggaagcaccg gaacgggtcg aatcaacccg 7380
   cgttgctgtt gctttggtga agcccgatga ttggaaactg attcagtctg atttcgcggt 7440
   taggcagtac gttctagatt gcgtggataa ggctaaggac gtggccgctg cgcaacgtgt 7500
   cgctaatgag gtgctggcaa gtctgggtgt ggattccacc ccgtgcatga tcgttatgga 7560
   tgatgtggac ttggacgcgg ttctgcctac tcatggggac gctactaagc gtgatctgaa 7620
   tgcggcggtg ttcgcgggta atgagcagac tattcttcgc acccactaaa agcggcataa 7680
   accccgttcg atattttgtg cgatgaattt atggtcaatg tcgcgggggc aaactatgat 7740
   gggtcttgtt gttgacaatg gctgatttca tcaggaatgg aactgtcatg ctgttatgtg 7800
   cctggctcct aatcaaagct ggggacaatg ggttgccccg ttgatctgat ctagttcgga 7860
   ttggcggggc ttcactgtat ctgggggtgg catcgtgaat agattgcaca ccgtagtggg 7920
   cagtgtgcac accatagtgg ccatgagcac caccaccccc agggacgccg acggcgcgaa 7980
   gctctgcgcc tggtgcggct cggagatcaa gcaatccggc gtcggccgga gccgggacta 8040
   ctgccgccgc tcctgccgcc agcgggcgta cgaggcccgg cgccagcgcg aggcgatcgt 8100
   gtccgccgtg gcgtcggcag tcgctcgccg agatacgtca cgtgacgaaa tgcagcagcc 8160
   ttccattccg tcacgtgacg aaactcgggc cgcaggtcag agcacggttc cgcccgctcc 8220
   ggccctgccg gacccccggc tgcagctcgc ccggccgccg gtccccctgc cgtccggccc 8280
   g 8281
<210> 30
   <211> 290
   <212> PRT
   <213> Corynebacterium glutamicum ATCC13032
<220>
   <221> misc_feature
   <222> (1)..(290)
   <223> Aminosäuresequenz des Aktivatorproteins LysG von Corynebacterium glutamicum ATCC13032
<400> 30

## Beschreibung

### Stand der Technik

L-Ornithin ist bekannt für seine stimulierende Wirkung für die Leberfunktion und wird häufig als ein Bestandteil von Medikamenten und in der Sporternährung genutzt.

L-Ornithin wird heute über verschiedene Verfahren hergestellt. Eine Methode ist die fermentative Herstellung mit Hilfe von Mikroorganismen. Eine weitere Methode ist die alkalische Hydrolyse von Arginin, z.B. mit Bariumhydroxid (CN 1594282 A). Eine weitere Methode ist die Biotransformation von Arginin durch immobilisierte Mikroorganismen, die eine Arginaseaktivität besitzen (KR589121B1). In der Patentliteratur ist ferner eine Methode zur Herstellung von L-Ornithin aus L-Citrullin beschrieben (JP 42007767 B4).

In der Literatur sind Mikroorganismen beschrieben, die sich dadurch auszeichnen, dass sie L-Ornithin ins Kulturmedium ausscheiden. Bei den Mikroorganismen handelt es sich z.B. um Bakterien der Gattung Corynebacterium, Brevibacterium, Bacillus (JP 43010996 B4, JP 57041912 B), Escherichia (US 3668072 A), Providencia (JP 03195494) oder Arthrobacter (US 3574061).

L-Ornithin produzierende Mikroorganismen zeichnen sich häufig dadurch aus, dass sie auxotroph für die Aminosäuren L-Arginin oder L-Citrullin sind (beschrieben für Brevibacterium, Bacillus, Corynebacterium in EP392708B1 und KR161147B1 und für Escherichia in US366072A). Weiterhin sind Mikroorganismen beschrieben die eine Resistenz gegenüber 2-Thiazole-Alanin, Sulfaguanidin oder 2-Fluor-Pyruvat besitzen (Japanische Offenlegungsschrift No 61-119194). In EP0393708B1 sind L-Ornithin Produzenten beschrieben, die sich durch eine geringere Resistenz gegenüber Ornithol und Mycophenolsäure auszeichnen. Die genannten Eigenschaften können auch miteinander kombiniert vorliegen.

Basische Aminosäuren wie L-Lysin, L-Arginin und L-Ornithin gelangen sehr schlecht per passiver Diffusion aus der Zelle (Bellmann et al. (Microbiology 2001; 147:1765-74)). Dies ist gut am Beispiel des Lysins beschrieben. Von Vrlijc et al. (Journal of Bacteriology 1995; 177(14):4021-7) wurden mehrere Export-defiziente Mutanten von Corynebacterium glutamicum untersucht. Bei einer Mutante wurde eine intrazelluläre Konzentration von 174 mM L-Lysin gemessen, während extrazellulär nur 0.7 mM gemessen werden konnten.

Von Vrlijc et al. (Molecular Microbiology 1996; 22(5):815-26 und Journal of Molecular Microbiology and Biotechnology 1999; 1:327-336) und in EP0868527B1 wurde ein neuartiger Exporter als L-Lysinexporter (LysE) identifiziert und beschrieben. Eine definierte LysE-Nullmutante war nicht mehr in der Lage L-Lysin aus der Zelle zu transportieren. Das vom lysE-Gen kodierte Polypeptid hat eine Länge von 233 Aminosäuren bzw. Aminosäureresten und ist in SEQ ID NO:2 wiedergegeben. Nach Überexpression des lysE-Gens in einem Lysinproduzenten wurde eine erhöhte Ausscheidung von L-Lysin verzeichnet.

Von Bellmann et al. (Microbiology 2001; 147:1765-74) wurde der Exporter LysE hinsichtlich des Transports verschiedener basischer Aminosäuren in C. glutamicum näher charakterisiert. Die Autoren zeigten, dass der Transporter spezifisch die Aminosäuren L-Lysin und L-Arginin aus der Zelle exportiert. Weiterhin gingen die Autoren der Frage nach, ob LysE auch L-Ornithin aus der Zelle exportiert. Dazu wurde zunächst ein als ATCC13032::argF bezeichneter, L-Arginin auxotropher C. glutamicum Stamm hergestellt.

Der Stamm wurde in 50 ml (batch-Kultur) eines als als CGXII bezeichneten Minimalmediums kultiviert, welches 40 g/l Glucose enthielt. Nach einer Inkubationszeit von 24 Stunden wurden 60 mM L-Ornithin, entsprechend 7,9 g/l, gemessen. Intrazellulär wurde in den Zellen des Stammes während einer Inkubationszeit von ca. 70 Minuten eine L-Ornithin Konzentration von ca. 200 mM gemessen. Um die Frage zu klären, ob LysE auch L-Ornithin aus der Zelle transportiert, wurde der Stamm 13032::argF mit dem replikativen Plasmid pEC7lysE transformiert. Durch diese Maßnahme sollte der Stamm eine erhöhte LysE-Aktivität erhalten und dadurch L-Ornithin mit einer höheren Exportrate ins Medium transportieren. Die L-Ornithin Exportrate wurde durch diese Maßnahme jedoch nicht erhöht. Sowohl beim Kontrollstamm (13032::argF) als auch in der Transformante (13032::argF enthaltend pEC7lysE) wurde dieselbe Exportrate (0,6 nmol min⁻¹ (mg Trockengewicht)⁻¹) bestimmt. Die Autoren schlossen daraus, dass L-Ornithin nicht durch den Exporter LysE exportiert wird. Sie folgerten weiterhin, dass es eine weitere, unbekannte Exportfunktion (Exportprotein) für L-Ornithin in Corynebacterium glutamicum geben muss (Bellmann et al., 2001, S. 1771, Fig.5b) bzw. S.1772 Z.21-28).

In C. glutamicum R wurde eine LysE-Variante (Siehe SEQ ID NO:4) identifiziert, die sich von der in SEQ ID NO:2 dargestellten Aminosäuresequenz des LysE-Exporters von Stamm ATCC 13032 durch einen um drei Aminosäurereste verlängerten N-Terminus unterscheidet. Bei den Aminosäureresten handelt es sich um die Abfolge Methionin, Valin, Isoleucin (MVI). Dieses LysE Polypeptid von Stamm R wurde in EP1266966B1 als Variante beschrieben, die sich vom Wildtypprotein in der Ausbildung einer Schleifenregion unterscheidet bzw. diese Schleife nicht mehr ausbilden kann und daher eine verbesserten Export von L-Lysin und L-Arginin bewerkstelligen kann.

Eine weitere Variante von LysE wurde von Gunji und Yasueda (Journal of Biotechnology 127, 2006, 1-13) beschrieben. Die Autoren waren an der L-Lysinbildung des obligat methylotrophen Bakteriums Methylophilus methylotrophus interessiert. Sie transformierten M. methylotrophus mit einem als pSE bezeichnetem Plasmid, welches das lysE-Gen aus C. glutamicum ATCC13869 enthielt, um die Lysinbildung von M. methylotrophus zu verbessern. Die Autoren fanden allerdings, dass sich nur eine mutierte Form des lysE-Gens (lysE24) stabil in M. methylotrophus etablieren ließ. Der offene Leseraster des lysE-Gens ist im lysE24-Allel durch die Insertion eines Thyminrestes verschoben, so dass es zu einem Leserasterabbruch nach 432 bp kommt. Der verkürzte Leseraster kodiert für ein gegenüber dem wildtypischen LysE-Protein von C. glutamicum ATCC13869 um 92 AS-Reste C-terminal verkürztes LysE-Protein. Es hat eine Länge von 141 Aminosäureresten. Die letzen 6 C-terminalen Aminosäuren des verkürzten Proteins (Reste 135-141) unterscheiden sich außerdem von den Aminosäuren der wildtypischen LysE-Aminosäuresequenz. Ein M. methylotrophus Stamm, der das veränderte LysE-Allel auf einem Plasmid trug (pSE24) wurde auf Lysinbildung geprüft. Hierzu wurde der Stamm für 50 Stunden in 0,3 1 eines als SEIIc bezeichneten Minimalmediums in Form einer fed-batch Kultur getestet.

Die Autoren fanden, dass die Transformante neben 0,55 mM L-Lysin und 0,19 mM L-Arginin auch geringe Mengen (0,07 mM entsprechend 11,8 mg/l) an L-Ornithin bildete. Die Autoren erklären dies mit einer entweder veränderten Substratspezifität des mutierten Transporters oder führen den veränderten intrazellulären L-Arginin-Pool des Stammes als mögliche Erklärung für die beobachtete L-Ornithin Bildung an. Im Patent EP1266966B1 (Erfinder Gunji und Yasueda) wird die positive Wirkung des LysE24 Transporters auf die L-Lysin und L-Argininausscheidung beschrieben.

### Aufgabe der Erfindung

Aufgabe der Erfindung ist die Bereitstellung eines neuen Verfahrens zur fermentativen Herstellung von L-Ornithin.

### Beschreibung der Erfindung

Gegenstand der Erfindung ist ein Verfahren zur Herstellung von L-Ornithin, dadurch gekennzeichnet, dass man folgende Schritte durchführt:
a) Fermentation eines L-Ornithin ausscheidenden Bakteriums, ausgewählt aus der Gruppe Corynebacterium, Bacillus, Streptomyces, Arthrobacter und der Enterobacteriaceae, in dem ein Polynukleotid, das für ein Polypeptid kodiert, welches die Aktivität eines L-Ornithin-Exporters hat und dessen Aminosäuresequenz mit SEQ ID NO: 2 identisch ist oder dessen Aminosäuresequenz durch insgesamt maximal 25 Deletionen, Insertionen, Substitutionen und/oder N- bzw. C-terminale Additionen von Aminosäuren aus SEQ ID NO: 2 erhalten werden kann, überexprimiert vorliegt, in einem Medium
b) Akkumulation des L-Ornithins in dem Medium, wobei eine Fermentationsbrühe erhalten wird,
c) wobei für die Uberexpression das Plasmid pEC7lysE hinterlegt in DSM23239 ausgeschlossen wird.

Wird im Folgenden L-Ornithin erwähnt, umfasst der Begriff auch dessen Salze wie beispielsweise das L-Ornithin-Monohydrochlorid oder L-Ornithin-Sulfat.

Für ein erfindungsgemäßes Verfahren werden Bakterien, ausgewählt aus der Gruppe Gattung Corynebacterium, Gattung

Bacillus, Gattung Streptomyces, Gattung Arthrobacter und der Familie Enterobacteriaceae verwendet.

Innerhalb der Gattung Corynebacterium werden Stämme bevorzugt, die auf folgenden Arten beruhen:
Corynebacterium efficiens, wie zum Beispiel der Typstamm DSM44549,
Corynebacterium glutamicum, wie zum Beispiel der Typstamm ATCC13032 oder der Stamm R, und
Corynebacterium ammoniagenes, wie zum Beispiel der Stamm ATCC6871,
wobei die Art Corynebacterium glutamicum ganz besonders bevorzugt wird.

Einige Vertreter der Art Corynebacterium glutamicum sind im Stand der Technik auch unter anderen Bezeichnungen bekannt. Hierzu gehören beispielsweise:
Stamm ATCC13870, der als Corynebacterium acetoacidophilum bezeichnet wurde,
Stamm DSM20137, der als Corynebacterium lilium bezeichnet wurde,
Stamm ATCC17965, der als Corynebacterium melassecola bezeichnet wurde,
Stamm ATCC14067, der als Brevibacterium flavum bezeichnet wurde,
Stamm ATCC13869, der als Brevibacterium lactofermentum bezeichnet wurde, und
Stamm ATCC14020, der als Brevibacterium divaricatum bezeichnet wurde.

Der Begriff "Micrococcus glutamicus" für Corynebacterium glutamicum war ebenfalls gebräuchlich. Einige Vertreter der Art Corynebacterium efficiens wurden im Stand der Technik auch als Corynebacterium thermoaminogenes bezeichnet, wie zum Beispiel der Stamm FERM BP-1539.

Innerhalb der Gattung Bacillus wird die Art Bacillus subtilis bevorzugt.

Innerhalb der Gattung Arthrobacter wird die Art Arthrobacter citreus bevorzugt.

Innerhalb der Familie der Enterobacteriacae werden die Gattungen Escherichia, Erwinia, Providencia, Pantoea und Serratia bevorzugt. Die Gattungen Escherichia und Serratia werden besonders bevorzugt. Bei der Gattung Escherichia wird die Art Escherichia coli, bei der Gattung Serratia wird die Art Serratia marcescens und bei der Gattung Providencia wird die Art Providencia rettgeri ganz besonders bevorzugt.

Die für die Maßnahmen der Überexpression des L-Ornithin-Exporters eingesetzten Bakterien bzw. Stämme (Ausgangsstämme) besitzen bevorzugt bereits die Fähigkeit L-Ornithin in das sie umgebende Nährmedium auszuscheiden und dort zu akkumulieren. Hierfür wird im Folgenden auch der Ausdruck "produzieren" verwendet. Insbesondere besitzen die für die Maßnahmen der Überexpression eingesetzten Stämme die Fähigkeit ≥ 0,1 g/l, ≥ 0,3 g/l, ≥ 1 g/l, ≥ 3 g/l, ≥ 10 g/l L-Ornithin im Nährmedium anzureichern bzw. zu akkumulieren. Bei den Ausgangsstämmen handelt es sich bevorzugt um Stämme, die durch Mutagenese und Selektion, durch rekombinante DNA-Techniken oder durch eine Kombination beider Methoden hergestellt wurden.

Es ist offensichtlich und bedarf keiner weiteren Erklärungen, dass man zu einem für die Maßnahmen der Erfindung geeignetem Bakterium auch dadurch gelangen kann, indem man in einem Wildstamm, wie zum Beispiel in dem Corynebacterium glutamicum Typstamm ATCC 13032 oder in dem Stamm ATCC 14067, zunächst ein Polynukleotid, das für ein Polypeptid kodiert, welches die Aktivität eines L-Ornithin-Exporters hat und dessen Aminosäuresequenz mit SEQ ID NO: 2 identisch ist oder dessen Aminosäuresequenz durch insgesamt maximal 25 Deletionen, Insertionen, Substitutionen und/oder N- bzw. C-terminale Additionen von Aminosäuren aus SEQ ID NO: 2 erhalten werden kann, überexprimiert und anschließend durch weitere, im Stand der Technik beschriebene, genetische Maßnahmen, das Bakterium veranlasst, L-Ornithin zu produzieren. Die Transformation eines Wildtyps, wie z. B. dem Stamm ATCC13032, ATCC14067, ATCC13869 oder ATCC17965, allein mit dem genannten Polynukleotid führt zu keinem erfindungsgemäßen Verfahren. L-Ornithin ausscheidende bzw. produzierende Stämme der Art Corynebacterium glutamicum sind beispielsweise:
Brevibacterium lactofermentum FERM-BP 2344, und
Corynebacterium glutamicum FERM-BP 2345 beschrieben in US5188947.

Ein L-Ornithin ausscheidender bzw. produzierender Stamm der Art Arthrobacter citreus ist beispielsweise:
Arthrobacter citreus FERM-BP 2342 beschrieben in US5188947.

Ein L-Ornithin ausscheidender bzw. produzierender Stamm der Art Bacillus subtilis ist beispielsweise:
Bacillus subtilis BOR-32 (FERM-P 3647) beschrieben in JP57041912.
Ein L-Ornithin ausscheidender bzw. produzierender Stamm der Art Providencia rettgeri ist beispielsweise:
   Providencia rettgeri ARGA6 (FERM P-11147) beschrieben JP03195494.

Ein L-Ornithin ausscheidender bzw. produzierender Stamm der Art Escherichia coli ist beispielsweise:
Escherichia coli B-19-19 (ATCC 21104) beschrieben in US3668072.

L-Ornithin produzierende Bakterien sind typischerweise auxotroph für die Aminosäuren L-Citrullin oder L-Arginin. Alternativ sind auch L-Ornithin produzierende Bakterien denkbar, die bradytroph für L-Citrullin oder L-Arginin sind. Definitionen der Begriffe auxotroph und bradytroph finden sich beispielsweise auf Seite 9 von WO 01/09286. Bradytrophe werden in der Fachwelt auch als leaky-Mutanten bezeichnet. Bei den bradytrophen Bakterien werden insbesondere solche verwendet bei denen die Aktivität der Genprodukte ArgF (Ornithin carbamoyltransferase), ArgG (Argininosuccinat Synthase) oder ArgH (Argininosuccinat Lyase) größer (>) Null aber kleinergleich (≤) 10 Prozent, bevorzugt > Null und ≤ 1 %, im Vergleich zur Aktivität im Wildtyp vorliegt.

Im Stand der Technik sind als lysE-Gen bezeichnete Polynukleotide bekannt, die für Proteine bzw. Polypeptide kodieren, die die Aktivität eines L-Lysinexporters besitzen. Diese Polypeptide werden auch als LysE abgekürzt.

Ein Exporter ist ein Protein, welches in der Zellmembran einer Zelle vorliegt und einen Metaboliten, z.B. L-Lysin oder L-Ornithin, aus dem Cytoplasma der Zelle heraus in das umgebende Medium transportiert. Wird die hierfür erforderliche Energie in Form von Adenosintriphosphat (ATP) bereitgestellt spricht man von primär aktivem Transport bzw. Export. Wird diese in Form eines Ionen-Gradienten von z.B. Natriumionen bereitgestellt spricht man von sekundär aktivem Transport bzw. Export (Jeremy M. Berg, John L. Tymoczko und L. Stryer; Biochemie, 5. Auflage, Seiten 378-384, Spektrum Akademischer Verlag, Heidelberg, Deutschland, 2003). Anleitungen zur Bestimmung der L-Ornithin Export Aktivität findet man bei Bellmann et al. (Microbiology 2001; 147:1765-74).

Bei den zur vorliegenden Erfindung führenden Arbeiten wurde gefunden, das die Lysinexporter der Gattungen Corynebacterium, bevorzugt Corynebacterium glutamicum, und Micrococcus, bevorzugt Micrococcus luteus, zusätzlich zur Aktivität des L-Lysinexportes die Aktivität eines L-Ornithinexporters besitzen.

Für die Maßnahmen der Erfindung werden Gene eingesetzt, die für Polypeptide mit Exportaktivität für L-Ornithin kodieren, deren Aminosäuresequenz mit SEQ ID NO: 2 identisch ist oder dessen Aminosäuresequenz durch insgesamt maximal 25 Deletionen, Insertionen, Substitutionen und/oder N- bzw. C-terminale Additionen von Aminosäuren aus SEQ ID NO: 2 erhalten werden kann.

Offenbarte L-Ornithinexporter sind beispielsweise die Lysinexporter bzw. LysE-Polypeptide von Corynebacterium glutamicum ATCC13032 (SEQ ID NO:2), Corynebacterium glutamicum R (SEQ ID NO:4), Corynebacterium glutamicum ATCC14067 (SEQ ID NO:5), Corynebacterium glutamicum ATCC13869 (SEQ ID NO:7), Corynebacterium efficiens YS-314 (SEQ ID NO:9), Corynebacterium diphteriae NCTC 13129 (SEQ ID NO:10), Corynebacterium striatum ATCC6940 (SEQ ID NO:11, Corynebacterium aurimucosum ATCC700975 (SEQ ID NO:12), Corynebacterium matruchotii ATCC33806 (SEQ ID NO:13), Corynebacterium pseudogenitalium ATCC33035 (SEQ ID NO:14), Corynebacterium accolens ATCC49725 (SEQ ID NO:15), Corynebacterium glucuronalyticum ATCC 51867 (SEQ ID NO:16), Micrococcus luteus NCTC2665 (SEQ ID NO:17), Corynebacterium tubuculostearicum SK141 (SEQ ID NO:18) und Corynebacterium matruchotii ATCC14266 (SEQ ID NO:19). SEQ ID NO:18 und SEQ ID NO:19 werden in der Fachwelt auch als ArgO-Polypeptide bezeichnet.

Die Nukleotidsequenz der lysE-Gene von Corynebacterium glutamicum ATCC14067 und Corynebacterium glutamicum ATCC13869 wurde in dieser Arbeit bestimmt (SEQ ID NO:6 und SEQ ID NO:8). Die Aminosäuresequenzen des LysE-Polypeptids von Corynebacterium glutamicum ATCC14067 und Corynebacterium glutamicum ATCC13869 sind in SEQ ID NO:5 und 7 dargestellt. Sie sind identisch zu der in SEQ ID NO:2 dargestellten Aminosäuresequenz des LysE von C. glutamicum ATCC13032.

In Tabelle 1 sind die Zugangsnummern (accession number), von LysE-Polypeptiden verschiedener Vertreter der Gattung Corynebacterium und von Micrococcus luteus aufgeführt, die den Datenbanken des National Center for Biotechnology Information (NCBI, Bethesda, MD, US) entnommen wurden. Weiterhin wird in Tabelle 1 auf die im Sequenzprotokoll wiedergegebenen Aminosäuresequenzen des LysE-Polypeptids verwiesen. Schließlich ist in Tabelle 1 die Länge (Anzahl der Aminosäuren) des kodierten LysE-Polypeptides angegeben.

**Tabelle 1**

| Bakterium | SEQ ID NO: | Zugangsnummer | Länge des Polypeptides |
|---|---|---|---|
| C. glutamicum | 2 | YP_225551.1 | 233 |
| C. efficiens | 9 | ZP_05749209.1 | 228 |
| C. diphteriae | 10 | NP_ 939452.1 | 228 |
| C. striatum | 11 | ZP_03933958.1 | 222 |
| C. aurimucosum | 12 | YP_ 002834652.1 | 235 |
| C. matruchotii | 13 | ZP_03711883.1 | 244 |
| C. pseudogenitalium | 14 | ZP_03922319.1 | 230 |
| C. accolens | 15 | ZP_03931790.1 | 241 |
| C. glucuronolyticum | 16 | ZP_03918361.1 | 261 |
| M. luteus | 17 | YP_002958101.1 | 204 |
| C. tubuculostearicum | 18 | ZP_05365683.1 | 230 |
| C. matruchotii | 19 | ZP_ 04835056.1 | 244 |

In Abbildung 1 ist ein Mehrfach-Vergleich (multiple sequence alignment) der Aminosäuresequenzen der LysE-Polypeptide der in Tabelle 1 angegebenen Bakterien dargestellt. Die in Abbildung 1 dargestellten Vergleiche der Aminosäuresequenzen wurden mit dem Programm Clone Manager 9 Professional Edition (Scientific & Educational Software 600 Pinner Weald Way Ste 202 Cary NC 27513 USA) angefertigt. Als Referenzmolekül für den Vergleich wurde das LysE-Polypeptid (LysE) von ATCC13032 verwendet. Für Treffermatrix (scoring matrix) wurde die Einstellung "Blosum 62" (Siehe: Jeremy M. Berg, John L. Tymoczko und L. Stryer; Biochemie, 5. Auflage, Seiten 194-197, Spektrum Akademischer Verlag, Heidelberg, Deutschland, 2003)) gewählt.

Gegebenenfalls können auch im Stand der Technik beschriebene Programme eingesetzt werden wie beispielsweise das Programm ClustalX (Thompson, J.D., Gibson,T.J., Plewniak,F., Jeanmougin,F. and Higgins,D.G. (1997). The ClustalX windows interface: flexible strategies for multiple sequence alignment aided by quality analysis tools. Nucleic Acids Research, 25:4876-4882).

Die Aminosäurereste 4 - 236 des LysE-Polypeptids von Corynebacterium glutamicum R (Siehe SEQ ID NO:4) entsprechen der in SEQ ID NO:2 dargestellten Aminosäuresequenz des LysE von C. glutamicum ATCC13032. Am N-Terminus besitzt das Polypeptid von C. glutamicum R zusätzlich eine Sequenz von drei Aminosäureresten (Methionin-Valin-Isoleucin). Diese zusätzlichen Reste ergeben sich, wenn alternativ zum Start-Codon des lysE-Gens in C. glutamicum ATCC13032 (siehe SEQ ID NO:1) das 9 Basenpaare weiter stromaufwärts vom lysE-Gen vorliegende Start-Codon verwendet wird.

Die Aminosäuresequenz des LysE-Polypeptids von C. efficiens YS-314 ist 71 % und die von C. diphteriae NCTC 13129 44%, die von Corynebacterium striatum ATCC6940 44%, die von Corynebacterium aurimucosum ATCC700975 42%, die von Corynebacterium matruchotii ATCC33806 43%, die von Corynebacterium pseudogenitalium ATCC33035 43%, die von Corynebacterium accolens ATCC49725 43%, die von Corynebacterium glucuronalyticum ATCC 51867 36%, die von Micrococcus luteus NCTC2665 40% identisch zu der in SEQ ID NO:2 dargestellten Aminosäuresequenz des LysE von C. glutamicum ATCC13032. Ferner ist die Aminosäuresequenz des ArgO-Polypeptids von C. tubuculostearicum SK141 zu 43% identisch zur Aminosäuresequenz von SEQ ID NO:2. Ferner ist die Aminosäuresequenz des ArgO-Polypeptids von C. matruchotii ATCC14266 zu 44% identisch zur Aminosäuresequenz von SEQ ID NO:2. Der Prozentsatz an Identität wurde durch Erstellung eines globalen Sequenzalignments mit Hilfe des Programmes Clone Manager 9 unter Verwendung der Einstellung Blosum 62 angefertigt (Siehe Abb. 2).

Die lysE-Gene, d. h. die Polynukleotide, die für Polypeptide mit der Aktivität eines L-Ornithin Exporters kodieren, können aus den Organismen mithilfe der Polymerase-Kettenreaktion (PCR) unter Verwendung geeigneter Primer isoliert werden. Anleitungen findet man unter anderem im Laborhandbuch "PCR" von Newton und Graham (Spektrum Akademischer Verlag, Heidelberg, Deutschland, 1994) und in der WO 2006/100211 auf Seite 14 bis 17.

Für ein erfindungsgemäßes Verfahren werden besonders bevorzugt Gene eingesetzt, die für Polypeptide mit L-Ornithin Export Aktivität kodieren, deren Aminosäuresequenz eines oder mehrere der Merkmale enthält ausgewählt aus der Gruppe
a) Aminosäuresequenz gemäß SEQ ID NO:2 oder SEQ ID NO:4,
b) Aminosäuresequenz gemäß SEQ ID NO:2 einschließlich eine oder mehrere, maximal 25, 20, 15, 10, 5, 4, 3, 2, oder 1 Deletion(en) von Aminosäuren,
c) Aminosäuresequenz gemäß SEQ ID NO:2 einschließlich eine oder mehrere, maximal 25, 20, 15, 10, 5, 4, 3, 2, oder 1 Insertion(en) von Aminosäuren, und
d) Aminosäuresequenz gemäß SEQ ID NO:2 einschließlich eine oder mehrere, maximal 25, 20, 15, 10, 5, 4, 3, 2, oder 1, bevorzugt maximal 5, 4, 3, 2, oder 1, Austausch(e) (Substitution(en)) von Aminosäuren.
e) Aminosäuresequenz gemäß SEQ ID NO:2 einschließlich eine oder mehrere maximal 25, 20, 15, 10, 5, 4, 3, 2,

### 1, bevorzugt maximal 5, 4, 3, 2, oder 1, Addition (en)

von Aminosäuren am N-Terminus und/oder am C-Terminus. Gegebenenfalls werden konservative Aminosäureaustausche bevorzugt. Bei den aromatischen Aminosäuren spricht man von konservativen Austauschen, wenn Phenylalanin, Tryptophan und Tyrosin gegeneinander ausgetauscht werden. Bei den hydrophoben Aminosäuren spricht man von konservativen Austauschen, wenn Leucin, Isoleucin und Valin gegeneinander ausgetauscht werden. Bei den polaren Aminosäuren spricht man von konservativen Austauschen, wenn Glutamin und Asparagin gegeneinander ausgetauscht werden. Bei den basischen Aminosäuren spricht man von konservativen Austauschen, wenn Arginin, Lysin und Histidin gegeneinander ausgetauscht werden. Bei den sauren Aminosäuren spricht man von konservativen Austauschen, wenn Asparaginsäure und Glutaminsäure gegeneinander ausgetauscht werden. Bei den Hydroxyl-Gruppen enthaltenden Aminosäuren spricht man von konservativen Austauschen, wenn Serin und Threonin gegeneinander ausgetauscht werden.

Offenbart werden Polynukleotide, die mit der zu SEQ ID NO:1, beispielsweise der Kodierregion von SEQ ID NO:1, komplementären Nukleotidsequenz unter stringenten Bedingungen hybridisieren und für ein Polypeptid mit L-Ornithin Export Aktivität kodieren, wobei die Aminosäuresequenz des kodierten Proteins ≥ 70% identisch ist mit der Aminosäuresequenz von SEQ ID NO:2 und wobei gegebenenfalls die Länge des kodierten Polypeptids sich in den oben beschriebenen Längenbereichen befindet. Anleitungen zur Hybridisierung von Nukleinsäuren beziehungsweise Polynukleotiden findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology 41: 255-260 (1991)). Die Hybridisierung findet unter stringenten Bedingungen statt, das heißt, es werden nur Hybride gebildet, bei denen die Sonde d.h. ein Polynukleotid umfassend die zu SEQ ID NO:1, beispielsweise die Kodierregion von SEQ ID NO:1, komplementäre Nukleotidsequenz und die Zielsequenz, d. h. die mit der Sonde behandelten beziehungsweise identifizierten Polynukleotide, mindestens 70% identisch sind. Es ist bekannt, dass die Stringenz der Hybridisierung einschließlich der Waschschritte durch Variieren der Pufferzusammensetzung, der Temperatur und der Salzkonzentration beeinflusst bzw. bestimmt wird. Die Hybridisierungsreaktion wird im Allgemeinen bei relativ niedriger Stringenz im Vergleich zu den Waschschritten durchgeführt (Hybaid Hybridisation Guide, Hybaid Limited, Teddington, UK, 1996).

Für die Hybridisierungsreaktion kann beispielsweise ein Puffer entsprechend 5x SSC-Puffer bei einer Temperatur von ca. 50°C - 68°C eingesetzt werden. Dabei können Sonden auch mit Polynukleotiden hybridisieren, die weniger als 70% Identität zur Nukleotidsequenz der eingesetzten Sonde aufweisen. Solche Hybride sind weniger stabil und werden durch Waschen unter stringenten Bedingungen entfernt. Dies kann beispielsweise durch Senken der Salzkonzentration auf 2x SSC oder 1x SSC und gegebenenfalls nachfolgend 0,5x SSC (The DIG System User's Guide for Filter Hybridisation, Boehringer Mannheim, Mannheim, Deutschland, 1995) erreicht werden, wobei eine Temperatur von ca. 50°C - 68°C, ca. 52°C - 68°C, ca. 54°C - 68°C, ca. 56°C - 68°C, ca. 58°C - 68°C, ca. 60°C - 68°C, ca. 62°C - 68°C, ca. 64°C - 68°C, ca. 66°C - 68°C eingestellt wird. Temperaturbereiche von ca. 64°C - 68°C oder ca. 66°C - 68°C werden bevorzugt. Es ist gegebenenfalls möglich, die Salzkonzentration bis auf eine Konzentration entsprechend 0,2x SSC oder 0,1x SSC zu senken. Gegebenenfalls enthält der SSC-Puffer Natriumdodecylsulfat (SDS) in einer Konzentration von 0,1%. Durch schrittweise Erhöhung der Hybridisierungstemperatur in Schritten von ca. 1 - 2°C von 50°C auf 68°C können Polynukleotidfragmente isoliert werden, die mindestens 70%, mindestens 80%, mindestens 90%, mindestens 92%, mindestens 94%, mindestens 96%, mindestens 97%, mindestens 98% oder mindestens 99%, gegebenenfalls 100% Identität zur Sequenz beziehungsweise komplementären Sequenz der eingesetzten Sonde besitzen und für ein Polypeptid mit L-Ornithin-Export-Aktivität kodieren. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog No. 1603558).

Für die Maßnahmen der Erfindung wird ein Polynukleotid in einem L-Ornithin produzierenden Bakterium bzw. Ausgangs- oder Elternstamm überexprimiert, welches für ein Protein kodiert, welches L-Ornithin Export Aktivität besitzt, wobei die Aminosäuresequenz mit SEQ ID NO: 2 identisch ist oder dessen Aminosäuresequenz durch insgesamt maximal 25 Deletionen, Insertionen, Substitutionen und/oder N- bzw. C-terminale Additionen von Aminosäuren aus SEQ ID NO: 2 erhalten werden kann.

Unter Überexpression versteht man allgemein eine Erhöhung der intrazellulären Konzentration oder Aktivität einer Ribonukleinsäure, eines Proteins (Polypeptids) oder eines Enzyms im Vergleich zum Ausgangsstamm (Elternstamm) oder Wildtypstamm, wenn dies der Ausgangsstamm ist. Unter einem Ausgangsstamm (Elternstamm) versteht man den Stamm, an dem die zur Überexpression führende Maßnahme durchgeführt wurde.

Die Begriffe Protein und Polypeptid werden als gegenseitig austauschbar betrachtet.

Bei der Überexpression werden die Methoden der rekombinanten Überexpression bevorzugt. Hierunter werden alle Methoden zusammengefasst bei denen ein Mikroorganismus unter Verwendung eines in-vitro bereitgestellten DNA-Moleküls hergestellt wird. Derartige DNA-Moleküle umfassen beispielsweise Promotoren, Expressionskassetten, Gene, Allele, Kodierregionen etc. Diese werden durch Methoden der Transformation, Konjugation, Transduktion oder gleichartige Methoden in den gewünschten Mikroorganismus überführt.

Durch die Maßnahmen der Überexpression, wird die Aktivität oder Konzentration des entsprechenden Polypeptids im Allgemeinen um mindestens 10%, 25%, 50%, 75%, 100%, 150%, 200%, 300%, 400% oder 500%, bevorzugt maximal bis 1000%, 2000%, 4000%, 10000% oder 20000% bezogen auf die Höhe der Aktivität oder Konzentration des Polypeptids im Stamm vor der zur Überexpression führenden Maßnahme, erhöht.

Gegebenenfalls ist bei Verwendung von Stämmen der Art Corynebacterium glutamicum die L-Ornithin-Exportaktivität in Stamm ATCC13032 oder ATCC14067 oder ATCC13869 oder ATCC17965 ein geeigneter Bezugspunkt zur Bestimmung der Überexpression. Bei Verwendung von Stämmen die auf ATCC13032 beruhen, bzw. ihren Ursprung haben ist der Stamm ATCC13032 ein geeigneter Bezugspunkt. Ein Beispiel hierfür ist der bei den zur vorliegenden Erfindung führenden Arbeiten hergestellte Stamm ATCC 13032_Delta_argFRGH/pVWEx1_lysE, der auf dem Stamm ATCC13032 beruht. Bei Verwendung von Stämmen die auf ATCC14067 beruhen, bzw. ihren Ursprung haben ist der Stamm ATCC14067 ein geeigneter Bezugspunkt. Bei Verwendung von Stämmen die auf ATCC13869 beruhen, bzw. ihren Ursprung haben ist der Stamm ATCC13869 ein geeigneter Bezugspunkt. Weitere geeignete Bezugspunkte ergeben sich entsprechender Weise.

Gegebenenfalls ist bei Verwendung von Stämmen der Art Escherichia coli, bevorzugt Escherichia coli Stamm K12 die L-Ornithin-Exportaktivität in Stamm MG1655 ein geeigneter Bezugspunkt zur Bestimmung der Überexpression.

Zur Erzielung einer Überexpression stehen im Stand der Technik eine Vielzahl von Methoden zur Verfügung.

Hierzu gehören die Erhöhung der Kopienzahl und die Modifikation der Nukleotidsequenzen, die die Expression des Gens steuern bzw. kontrollieren. Die Transkription eines Gens wird unter anderem kontrolliert durch den Promotor und gegebenenfalls durch Proteine, welche die Transkription unterdrücken (Repressorproteine) oder fördern (Aktivatorproteine). Die Translation der gebildeten RNA wird unter anderem kontrolliert durch die Ribosomenbindungsstelle und das Startkodon. Polynukleotide bzw. DNA-Moleküle, welche einen Promotor und eine Ribosomenbindungsstelle und gegebenenfalls ein Startkodon umfassen, werden auch als Expressionskassette bezeichnet.

Hierzu gehört auch der Einsatz von Varianten von Polypeptiden bzw. Enzymen, die eine erhöhte katalytische Aktivität aufweisen.

Die Erhöhung der Kopienzahl kann durch Plasmide erfolgen, die im Cytoplasma des Bakteriums replizieren. Hierzu sind im Stand der Technik eine Fülle von Plasmiden für die verschiedensten Gruppen von Mikroorganismen beschrieben, mit denen man die gewünschte Erhöhung der Kopienzahl des Gens einstellen kann. Geeignete Plasmide für die Gattung Escherichia sind beispielsweise im Handbuch Molecular Biology, Labfax (Ed.: T. A. Brown, Bios Scientific, Oxford, UK, 1991) beschrieben. Geeignete Plasmide für die Gattung Corynebacterium sind beispielsweise bei Tauch et al. (Journal of Biotechnology 104 (1-3), 27-40, (2003)), oder bei Stansen et al. (Applied and Environmental Microbiology 71, 5920-5928 (2005)) beschrieben.

Die Verwendung des Plasmids pEC7lysE hinterlegt in DSM 23239 zur Erhöhung der Kopienzahl in Corynebacterium glutamicum Stämmen wird von den zur Erfindung führenden Maßnahmen ausgeschlossen. Die Nukleotidsequenz des Plasmids pEC7lysE wurde bestimmt und ist in SEQ ID NO:29 dargestellt.

Die Erhöhung der Kopienzahl um mindestens eine (1) Kopie kann weiterhin durch Einfügen weiterer Kopien in das Chromosom des Bakteriums erfolgen. Geeignete Methoden für die Gattung Corynebacterium, bevorzugt Corynebacterium glutamicum, sind beispielsweise in der Patentschrift WO 03/014330, WO 03/040373 und WO 04/069996 beschrieben. In der WO 03/014330 sind Methoden zur Tandem-Verdopplung von Genen am nativen Genort beschrieben. In der WO 03/040373 sind Methoden beschrieben um eine zweite oder dritte Kopie eines Gens in weitere Genorte einzubauen, wobei der jeweilige Genort nicht essentiell für Wachstum oder Produktion der jeweiligen Aminosäure, im Falle der vorliegenden Erfindung L-Ornithin, ist. Geeignete Genorte zum Einbau einer zweiten bzw. weiteren Kopie des lysE-Gens in einem erfindungsgemäßen Verfahren sind beispielsweise die Gene odh, sucA, dapA, dapB, ddh, lysA, argR, argF, argG und argH. In der WO 04/069996 (siehe Tabelle 12 und 13) sind intergenische Regionen und Gene, die für Phagen beziehungsweise Phagenkomponenten kodieren, von C. glutamicum beschrieben, welche für den Einbau weiterer Kopien des lysE-Gens geeignet sind.

Geeignete Methoden für die Gattung Escherichia sind beispielsweise der Einbau einer Genkopie in die att-site des Phagen (Yu und Court, Gene 223, 77-81 (1998)), die chromosomale Amplifikation mit Hilfe des Phagen Mu, wie in der EP 0 332 448 beschrieben, oder die von Hamilton et al. (Journal of Bacteriology 174, 4617 - 4622 (1989)) oder Link et al. (Journal of Bacteriology 179, 6228-6237 (1997)) beschriebenen Methoden des Genaustausches mit Hilfe konditional replizierender Plasmide.

Die Erhöhung der Genexpression kann weiterhin durch Verwendung eines starken Promotors erzielt werden, der funktionell mit dem zu exprimierenden Gen verknüpft wird. Vorzugsweise wird ein Promotor verwendet, der stärker ist als der natürliche, d. h. der im Wildtyp oder Elternstamm vorhandene Promotor. Hierfür stehen im Stand der Technik eine Fülle von Methoden zur Verfügung.

Geeignete Promotoren und Expressionssysteme für die Gattung Corynebacterium finden sich unter anderem in den Patentschriften EP 0 629 699 A2, US 2007/0259408 A1 (gap-Promotor), WO 2006/069711, EP 1 881 076 A1, WO 2008/088158, WO 2009/025470 (butA-Promotor, pyk-Promotor), US 6,861,246 (MC20- und MA16-Variante des dapA-Promotors) und EP 1 918 378 A1 (sod-Promotor) und in zusammenfassenden Darstellungen wie dem "Handbook of Corynebacterium glutamicum" (Eds.: Lothar Eggeling und Michael Bott, CRC Press, Boca Raton, US (2005)) oder dem Buch "Corynebacteria, Genomics and Molecular Biology" (Ed.:Andreas Burkovski, Caister Academic Press, Norfolk, UK (2008)). Beispiele für Promotoren, die eine kontrollierte, das heißt induzierbare oder reprimierbare Expression erlauben, sind beispielsweise bei Tsuchiya und Morinaga (Bio/Technology 6, 428-430 (1988)) beschrieben.

Geeignete Promotoren für die Gattung Escherichia sind seit langem bekannt. Hierzu gehören unter anderem die klassischen Promotoren lac-Promotor, trp-Promotor, die Hybrid-Promotoren tac und trc, die Promotoren PL und PR des Phagen λ. In gleicher Weise können die Promotoren des Phagen T7, die gear-box-Promotoren, der nar-Promotor oder die Promotoren der Gene rrsG, rnpB, csrA, csrB, ompA, fusA, pepQ, rplX oder rpsG verwendet werden. Eine kontrollierte Expression erlaubt beispielsweise das cI857-PR- oder das cI857-PL-System des Phagen λ (Götting et al., BioTechniques 24, 362-366 (1998)). Zusammenfassende Darstellungen finden sich bei Makrides (Microbiological Reviews 60(3), 512-538 (1996)) oder dem Handbuch "Escherichia coli and Salmonella, Cellular and Molecular Biology" (F. C. Neidhardt (Editor in Chief), ASM Press, Washington, US (1996)).

Derartige Promotoren oder Expressionskassetten werden typischerweise im Abstand von 1 bis 1000, bevorzugt 1 bis 500, Nukleotiden stromaufwärts des ersten Nukleotids des Startkodons der Kodierregion des Gens eingesetzt. Im Abstand von 1 bedeutet, dass der Promotor oder die Expressionskassette direkt vor die erste Base des Startkodons der Kodierregion positioniert wird.

Zur Steigerung der Expression des lysE-Gens in C. glutamicum werden geeignete Promotoren wie beispielsweise der sod-Promotor von C. glutamicum (siehe SEQ ID NO:1 von EP 1918 378 A1) oder der gap-Promotor von C. glutamicum (siehe SEQ NO:3 von US 2007/0259408) bevorzugt zwischen Position 930 und 990 von SEQ ID NO:1 eingefügt.

Bei Verwendung von Expressionskassetten, enthaltend einen Promotor und eine Ribosomenbindungsstelle (RBS), wie beispielsweise die Expressionseinheit des sod-Gens von C. glutamicum (siehe SEQ ID NO:2 von EP 1918 378 A1) oder die Expressionseinheit des gap-Gens von C. glutamicum, beschrieben in US 2007/0259408 und wiedergegeben in SEQ ID NO:28 (und dort bezeichnet als PgapRBS), werden diese im Falle von C. glutamicum bevorzugt zwischen Position 930 und 1001, besonders bevorzugt zwischen Position 1000 und 1001 von SEQ ID NO:1 eingefügt. Eine geeignete Ribosomenbindestelle in einer derartigen Expressionskassette ist beispielsweise die von Amador (Microbiology 145, 915-924 (1999)) angegebene Nukloeotidsequenz 5'-agaaaggagg-3'.

Es ist ebenfalls möglich, mehrere Promotoren vor das gewünschte Gen zu positionieren bzw. funktionell mit dem zum exprimierenden Gen zu verknüpfen und auf diese Weise zu einer erhöhten Expression zu gelangen. Dies ist beispielsweise in der WO 2006/069711 beschrieben.

Die Struktur von Promotoren von Corynebacterium glutamicum und Escherichia coli ist gut bekannt. Es ist daher möglich, die Stärke eines Promotors durch Modifikation seiner Sequenz mittels einem oder mehreren Austausch(en) und/oder einer oder mehrerer Insertion(en) und/oder einer oder mehrerer Deletion(en) von Nukleotiden zu erhöhen. Beispiele hierfür finden sich unter anderem in "Herder Lexikon der Biologie" (Spektrum Akademischer Verlag, Heidelberg, Deutschland (1994)).

Eine geeignete Maßnahme zur Überexpression des lysE-Gens ist dementsprechend die Modifikation bzw. Mutation des Promotors des lysE-Gens.

Die Struktur der Ribosomenbindungsstelle von Corynebacterium glutamicum und von Escherichia coli ist ebenfalls gut bekannt und beispielsweise bei Amador (Microbiology 145, 915-924 (1999)) und in Hand- und Lehrbüchern der Genetik, wie beispielsweise "Gene und Klone" (Winnacker, Verlag Chemie, Weinheim, Deutschland (1990)) oder "Molecular Genetics of Bacteria" (Dale und Park, Wiley and Sons Ltd., Chichester, UK (2004)) beschrieben. Gut exprimierte Gene, d.h. die wichtigsten Strukturgene in einem Organismus, verfügen über eine gute Ribosomenbindestelle (Amador, Microbiology 145, 915-924 (1999)), d.h. diese hat eine hohe Ähnlichkeit zur oder entspricht der Consensussequenz. Es wurde in der Literatur gezeigt, dass hochexprimierte Gene eine starke Ribosomenbindestelle aufweisen (Karlin and Mräzek, Journal of Bacteriology 2000; 182(18):5238-50). Die Translationseffizienz eines Gens bzw. der m-RNA kann deshalb durch Anpassung der Ribosomenbindestelle erreicht werden.

Eine weitere Möglichkeit die Translationseffizienz zu steigern, ist die Anpassung der Codon-Usage in den zu exprimierenden Genen (z.B. Najafabiad et al.; Nucleic Acids Research 2009, 37 (21) : 7014-7023).

Zur Erzielung einer Überexpression ist es ebenfalls möglich, die Expression von Aktivatorproteinen zu steigern oder die Expression von Repressorproteinen zu verringern oder auszuschalten.

Das Aktivatorprotein LysG für die Expression von lysE wurde von Bellmann et al. (Microbiology 2001; 147:1765-74) beschrieben. Es wird dort als "positive regulator" bezeichnet. Die Aminosäuresequenz von LysG von Corynebacterium glutamicum ATCC13032 ist in SEQ ID NO:30 wiedergegeben. In einem globalen Sequenzalignment ist die Aminosäuresequenz des LysG-Polypeptids von Corynebacterium diphteriae NCTC13129 zu 62%, die Aminosäuresequenz des LysG-Polypeptids von Corynebacterium efficiens YS-314 zu 81% und die Aminosäuresequenz des LysG-Polypeptids Von Corynebacterium glutamicum R 94% identisch mit der von SEQ ID NO:30.

Bei den Aktivatorproteinen wird ein Polypeptid bevorzugt, welches ≥ (mindestens) 55%, bevorzugt ≥ 80%, besonders bevorzugt ≥ 90%, ≥ 92% oder ≥ 94 %, ganz besonders bevorzugt ≥ 99% und äußerst bevorzugt 100 % identisch ist mit der in SEQ ID NO:30 dargestellten Aminosäuresequenz.

Die genannten Maßnahmen zur Überexpression, bevorzugt ausgewählt aus der Gruppe Erhöhung der Kopienzahl, Verwendung eines starken Promotors, Mutation des Promotors, Verwendung einer geeigneten Expressionskassette und Überexpression eines Aktivatorproteins, können in geeigneter Weise miteinander kombiniert werden. So kann beispielsweise die Verwendung eines geeigneten Promotors mit der Erhöhung der Kopienzahl oder die Überexpression eines Aktivatorproteins kann mit der Verwendung eines geeigneten Promotors oder einer geeigneten Expressionskassette kombiniert werden.

Es ist ebenfalls möglich, zusätzlich zu den Maßnahmen die das Polynukleotid betreffen, welches für ein Protein mit L-Ornithin Export Aktivität kodiert, einzelne Biosynthesegene abzuschwächen.

So ist es gegebenenfalls zweckmäßig für die Verbesserung der Produktion von L-Ornithin, zusätzlich eines oder mehrere der Gene ausgewählt aus der Gruppe
a) odhA-Gen, das für die E1-Untereinheit der alpha-Ketoglutarat-Dehydrogenase (EC 1.2.4.2) kodiert,
b) sucA-Gen, das für die Dihydrolipoamide Succinyltransferase (EC 2.3.1.61) kodiert,
c) dapA-Gen, das für eine Dihydrodipicolinat-Synthase (DapA, EC 4.2.1.52) kodiert,
d) dapB-Gen, das für eine Dihydrodipicolinat-Synthase (DapB, EC 1.3.1.26) kodiert,
e) ddh-Gen, das für eine meso-Diaminopimelat Dehydrogenase (Ddh, EC 1.4.1.16) kodiert,
f) lysA-Gen, das für eine Diaminopimelat-Decarboxylase (LysA, EC 4.1.1.20) kodiert,
g) argR-Gen, das für einen/den Repressor (ArgR) der Biosynthese von L-Arginin kodiert,
h) argF-Gen, das für eine Ornitin Carbamoyl Transferase (ArgF, EC 2.1.3.3) kodiert,
i) argG-Gen, das für eine Argininosuccinat Synthase (ArgG, EC 6.3.4.5) kodiert,
j) argH-Gen, das für eine Argininosuccinat Lyase (ASAL) (ArgH, EC 4.3.2.1) kodiert,
k) lysC-Gen, das für eine Aspartatkinase (LysC, EC 2.7.2.4) kodiert, und
l) asd-Gen, das für eine Aspartatsemialdehyd-Dehydrogense (Asd, EC 1.2.1.11) kodiert.
abzuschwächen.

Bevorzugt wird die Abschwächung eines oder mehrerer der Gene ausgewählt aus der Gruppe lysA, odhA, argR argF, argG und argH. Besonders bevorzugt wird die Abschwächung eines oder mehrerer der Gene ausgewählt aus der Gruppe lysA, odhA und argF. Ganz besonders bevorzugt wird die Abschwächung der Gene lysA und/oder argF.

Der Begriff "Abschwächung" beschreibt in diesem Zusammenhang die Verringerung oder Ausschaltung der intrazellulären Aktivität eines oder mehrerer Enzyme (Proteine) in einem Bakterium, die durch die entsprechende DNA kodiert werden, indem man beispielsweise einen schwachen Promotor verwendet oder ein Gen bzw. Allel verwendet, das für ein entsprechendes Enzym mit einer niedrigen Aktivität kodiert bzw. das entsprechende Gen oder Enzym (Protein) inaktiviert und gegebenenfalls diese Maßnahmen kombiniert.

Eine Übersicht über bekannte Promotoren verschiedener Stärke in Corynebacterium glutamicum findet man bei Pátek et al. (Journal of Biotechnology 104, 311-323 (2003)). Weitere schwache Promotoren sind in der Mitteilung 512057 der Zeitschrift Research Disclosure vom Dezember 2006 (Seiten 1616 bis 1618) beschrieben.

Als Mutationen zur Erzeugung einer Abschwächung kommen Transitionen, Transversionen, Insertionen und Deletionen von mindestens einem (1) Basenpaar bzw. Nukleotid in der Kodierregion des betreffenden Gens in Betracht. In Abhängigkeit von der Wirkung des durch die Mutation hervorgerufenen Aminosäureaustausches auf die Aktivität des Proteins bzw. Enzyms wird von Fehlsinnmutationen ("missense mutations") oder Nichtsinnmutationen ("nonsense mutations") gesprochen.

Die Fehlsinnmutation führt zu einem Austausch einer gegebenen Aminosäure in einem Protein gegen eine andere, wobei es sich insbesondere um einen nicht-konservativen Aminosäureaustausch handelt. Hierdurch wird die Funktionsfähigkeit bzw. Aktivität des Proteins beeinträchtigt und auf einen Wert von ≥ 0 bis 75%, ≥ 0 bis 50%, ≥ 0 bis 25%, ≥ 0 bis 10% oder ≥ 0 bis 5% reduziert.

Die Nichtsinnmutation führt zu einem Stopp-Kodon im Kodierbereich des Gens und damit zu einem vorzeitigen Abbruch der Translation und somit zu einer Ausschaltung. Insertionen oder Deletionen von mindestens einem Basenpaar in einem Gen führen zu Rasterverschiebungsmutationen ("frame shift mutations"), die dazu führen, dass falsche Aminosäuren eingebaut werden oder die Translation vorzeitig abbricht. Entsteht als Folge der Mutation ein Stopp-Kodon im Kodierbereich, so führt dies ebenfalls zu einem vorzeitigen Abbruch der Translation. Die Maßnahmen zur Erzeugung einer Nichtsinnmutation werden vorzugsweise im 5'-terminalen Teil der Kodierregion durchgeführt, welcher für den N-Terminus des Polypeptids kodiert. Bezeichnet man die Gesamtlänge eines Polypeptids (gemessen als Anzahl der chemisch verbundenen L-Aminosäuren) mit 100%, so gehört - im Rahmen der vorliegenden Erfindung - zum N-Terminus des Polypeptids der Teil der Aminosäuresequenz, welcher, gerechnet ab der Start-Aminosäure L-Formyl-Methionin 80% der nachfolgenden L-Aminosäuren enthält.

In-vivo Mutagenesemethoden sind beispielsweise im Manual of Methods for General Bacteriology (Gerhard et al. (Eds.), American Society for Microbiology, Washington, DC, USA, 1981) oder bei Tosaka et al. (Agricultural and Biological Chemistry 42(4), 745-752 (1978)) oder bei Konicek et al. (Folia Microbiologica 33, 337-343 (1988)) beschrieben.

Geeignete Methoden für die in-vitro Mutagenese sind unter anderem die Behandlung mit Hydroxylamin nach Miller (Miller, J. H.: A Short Course in Bacterial Genetics. A Laboratory Manual and Handbook for Escherichia coli and OxyRated Bacteria, Cold Spring Harbor Laboratory Press, Cold Spring Harbor, 1992), die Verwendung mutagener Oligonukleotide (T. A. Brown: Gentechnologie für Einsteiger, Spektrum Akademischer Verlag, Heidelberg, 1993 und R. M. Horton: PCR-Mediated Recombination and Mutagenesis, Molecular Biotechnology 3, 93-99 (1995)) und der Einsatz einer Polymerasekettenreaktion unter Verwendung einer DNA-Polymerase, die eine hohe Fehlerquote aufweist. Eine derartige DNA-Polymerase ist beispielsweise die Mutazyme DNA Polymerase (GeneMorph PCR Mutagenesis Kit, Nr.600550) der Firma Stratagene (LaJolla, CA, USA).

Weitere Anleitungen und Übersichten zur Erzeugung von Mutationen in-vivo oder in-vitro können dem Stand der Technik und bekannten Lehrbüchern der Genetik und Molekularbiologie wie z.B. dem Lehrbuch von Knippers ("Molekulare Genetik", 6. Auflage, Georg Thieme Verlag, Stuttgart, Deutschland, 1995), dem von Winnacker ("Gene und Klone", VCH Verlagsgesellschaft, Weinheim, Deutschland, 1990) oder dem von Hagemann ("Allgemeine Genetik", Gustav Fischer Verlag, Stuttgart, 1986) entnommen werden.

Mit Hilfe des bekannten Verfahren des Gen- bzw. Allel-Austauschs, das in seinen Grundzügen bei Schwarzer und Pühler (Bio/Technology 9, 84-87 (1991)) beschrieben ist kann eine in-vitro hergestellte Mutation, bzw. ein Polynukleotid enthaltend die gewünschte Mutation in das Chromosom überführt werden. Von Schäfer et al. (Gene 145, 69-73 (1994)) wurde diese Methode eingesetzt, um eine Deletion in das hom-thrB Operon von C. glutamicum einzubauen. Von Nakagawa et al. (EP 1108790) und Ohnishi et al. (Applied Microbiology and Biotechnology 58(2), 217-223 (2002)) wurde diese Methode eingesetzt, um verschiedene Mutationen ausgehend von den isolierten Allelen in das Chromosom von C. glutamicum einzubauen.

Ein Methode zur gezielten Verringerung der Genexpression besteht darin, das abzuschwächende Gen unter die Kontrolle eines durch Zugabe dosierter Mengen von IPTG (Isopropyl-ß-D-thiogalactopyranosid) induzierbaren Promotors, wie zum Beispiel des trc-Promotors oder des tac-Promotors, zu stellen. Hierzu eignen sich Vektoren wie beispielsweise der Escherichia coli Expressionsvektor pXK99E (WO0226787; hinterlegt gemäß Budapester Vertrag am 31. Juli 2001 in DH5alpha/pXK99E als DSM14440 bei der Deutschen Sammlung für Mikroorganismen und Zellkulturen (DSMZ, Braunschweig, Deutschland)), pEKEx2 (NCBI Accession No. AY585307) oder pVWEx2 (Wendisch, Ph. D. thesis, Berichte des Forschungszentrums Jülich, Jül-3397, ISSN 0994-2952, Jülich, Deutschland (1997)), die eine IPTG-abhängige Expression des klonierten Gens in Corynebacterium glutamicum ermöglichen.

Eingesetzt wurde diese Methode beispielsweise in der Patentschrift WO0226787 zur regulierten Expression des deaD-Gens durch Integration des Vektors pXK99EdeaD in das Genom von Corynebacterium glutamicum und von Simic et al. (Applied and Environmental Microbiology 68: 3321-3327 (2002)) zur regulierten Expression des glyA-Gens durch Integration des Vektors pK18mobglyA' in Corynebacterium glutamicum.

Eine weitere Methode zur spezifischen Verringerung der Genexpression ist die Antisense-Technik, wobei kurze Oligodesoxynukleotide oder Vektoren zur Synthese längerer Antisense-RNA in die Zielzellen gebracht werden. Die Antisense-RNA kann dort an komplementäre Abschnitte spezifischer mRNAs binden und deren Stabilität verringern oder die Translatierbarkeit blocken. Ein Beispiel hierzu findet der Fachmann bei Srivastava et al. (Applied Environmental Microbiology 2000 Oct; 66 (10): 4366 - 4371).

Die Geschwindigkeit der Elongation wird durch die Kodon-Verwendung beeinflusst. Durch die Verwendung von Kodons für im Elternstamm (parent strain) selten vorkommenden t-RNAs kann die Genexpression abgeschwächt werden. Dies ist ausführlich in der WO2008049781 und in der WO2009133063 beschrieben. Beispielsweise kann der Austausch eines ATG Start-Kodons zu den seltener vorkommenden Kodons GTG oder TTG die Translation verschlechtern, da das Kodon AUG zweibis dreimal effektiver ist als zum Beispiel die Kodons GUG und UUG (Khudyakov et al., FEBS Letters 232(2):369-71 (1988); Reddy et al., Proceedings of the National Academy of Sciences of the USA 82(17):5656-60 (1985)).

Es ist ebenfalls möglich, zusätzlich zu den Maßnahmen die das Polynukleotid betreffen, welches für ein Protein mit L-Ornithin Export Aktivität kodiert, einzelne Biosynthesegene zu verstärken.

So ist es gegebenenfalls zweckmäßig für die Verbesserung der Produktion von L-Ornithin, zusätzlich die Enzymaktivität eines oder mehrere der Proteine ausgewählt aus der Gruppe
a) Glutamat-Dehydrogenase (EC 1.4.1.3) kodiert vom gdh-Gen,
b) Glutamat N-Acetyltransferase (EC 2.3.1.35 und EC 2.3.1.1) kodiert vom argJ-Gen,
c) Acetylglutamatkinase (EC 2.7.2.8) kodiert vom argB-Gen,
d) N-Acetyl-Gamma-Glutamyl-Phosphat-Reduktase (EC 1.2.1.38) kodiert vom argC-Gen,
e) Acetyl-Ornithin Aminotransferase (EC 2.6.1.11), kodiert vom argD-Gen,
f) Glukose spezifische Komponente EIIB (PtsG) (EC 2.7.1.69) des Glukose-Aufnahmesystems kodiert vom ptsG-Gen,
g) Saccharose spezifische Komponente EIIB (PtsS) (EC 2.7.1.69) des Saccharose-Aufnahmesystems kodiert vom ptsS-Gen,
h) Glucose-6-Phosphat 1- Dehydrogenase (EC 1.1.1.49) kodiert vom zwf-Gen,
i) Glucose-6-Phosphat-Isomerase (EC 5.3.1.9) kodiert vom pgi-Gen,
j) Phoshofructokinase (EC 2.7.1.11) kodiert vom pfkA-Gen,
k) Fructose-bisphosphat aldolase (EC 4.1.2.13) kodiert vom fda-Gen,
l) Glyceraldehyd-3-Phosphat Dehydrogenase (EC 1.2.1.59), kodiert vom gap-Gen,
m) Phosphoglycerat Kinase (EC 2.7.2.3) kodiert vom pgk-Gen,
n) Pyruvatkinase (EC 2.7.1.40) kodiert vom pyk-Gen,
o) E1-Untereinheit der Pyruvat-Dehydrogenase (EC 1.2.4.1) kodiert vom aceE-Gen,
p) Phosphoenolpyruvat-Carboxylase (EC 4.1.1.31) kodiert vom ppc-Gen,
q) Pyruvat-Carboxylase (EC 6.4.1.1), kodiert vom pyc-Gen,
r) Aconitase (EC 4.2.1.3) kodiert vom acn-Gen, und
s) Isocitrat-Dehydrogenase (EC 1.1.1.42) kodiert vom icd-Gen
zu verstärken.

Der Begriff Verstärkung umfasst die Massnahmen zur Überexpression und die Verwendung von Varianten mit erhöhter katalytischer Aktivität im Vergleich zum Protein des Wildtyps.

Besonders bevorzugt wird die Verstärkung eines oder mehrerer der Enzyme ausgewählt aus der Gruppe Glutamat-Dehydrogenase, Glutamat N-Acetyltransferase und der Acetylglutamatkinase.

Die aufgeführten Zusatzmaßnahmen der Abschwächung können mit den Zusatzmaßnahmen der Verstärkung kombiniert werden.

Anleitungen zum Umgang mit DNA, Verdauung und Ligation von DNA, Transformation und Selektion von Transformanten findet man unter anderem in dem bekannten Handbuch von Sambrook et al. "Molecular Cloning: A Laboratory Manual, Second Edition (Cold Spring Harbor Laboratory Press, 1989).

Das Ausmaß der Expression beziehungsweise Überexpression kann durch Messung der Menge bzw. Konzentration der vom Gen transkribierten mRNA, durch Bestimmung der Menge bzw. Konzentration des Polypeptids und durch Bestimmung der Höhe der Enzymaktivität festgestellt werden.

Für die Bestimmung der Menge an mRNA können unter anderem die Methode des "Northern Blotting's" und der quantitativen RT-PCR verwendet werden. Bei der quantitativen RT-PCR wird der Polymerase-Kettenreaktion eine reverse Transkription vorgeschaltet. Hierzu kann das LightCyclerTM System der Firma Roche Diagnostics (Boehringer Mannheim GmbH, Roche Molecular Biochemicals, Mannheim, Deutschland) verwendet werden, wie beispielsweisae bei Jungwirth et al. (FEMS Microbiology Letters 281, 190-197 (2008)) beschrieben. Die Konzentration des Proteins kann über 1- und 2-dimensionale Proteingelauftrennung und anschließende optische Identifizierung der Proteinkonzentration mit enstprechender Auswertesoftware im Gel bestimmt werden. Eine gebräuchliche Methode zur Präparation der Proteingele bei coryneformen Bakterien und zur Identifizierung der Proteine ist die von Hermann et al. (Electrophoresis, 22:1712-23 (2001)) beschriebene Vorgehensweise. Die Proteinkonzentration kann ebenfalls durch Western-Blot-Hybridisierung mit einem für das nachzuweisende Protein spezifischen Antikörper (Sambrook et al., Molecular cloning: a laboratory manual. 2nd Ed. Cold Spring Harbor Laboratory Press, Cold Spring Harbor, N.Y., 1989) und anschließender optischer Auswertung mit ensprechender Software zur Konzentrationsbestimmung (Lohaus und Meyer (1998) Biospektrum 5:32-39; Lottspeich, Angewandte Chemie 321: 2630-2647 (1999)) bestimmt werden.

Die hergestellten Bakterien können kontinuierlich - wie beispielsweise in der WO 05/021772 beschrieben- oder diskontinuierlich im batch - Verfahren (Satzkultivierung bzw. Satzverfahren) oder im fed batch (Zulaufverfahren) oder repeated fed batch Verfahren (repetitives Zulaufverfahren, beispielsweise beschrieben in US 6,562,601) zum Zwecke der Produktion von L-Ornithin kultiviert werden. Eine Zusammenfassung allgemeiner Art über bekannte Kultivierungsmethoden ist im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) verfügbar.

Das zu verwendende Kulturmedium beziehungsweise Fermentationsmedium muß in geeigneter Weise den Anforderungen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten. Die Begriffe Nährmedium, Kulturmedium und Fermentationsmedium beziehungsweise Medium sind gegenseitig austauschbar.

Als Kohlenstoffquelle können Zucker und Kohlehydrate wie z.B. Glucose, Saccharose, Lactose, Fructose, Maltose, Melasse, Saccharose-haltige Lösungen aus der Zuckerrüben- oder Zuckerrohrverarbeitung, Stärke, Stärkehydrolysat und Cellulose, Öle und Fette, wie zum Beispiel Sojaöl, Sonnenblumenöl, Erdnußöl und Kokosfett, Fettsäuren, wie zum Beispiel Palmitinsäure, Stearinsäure und Linolsäure, Alkohole wie zum Beispiel Glycerin, Methanol und Ethanol und organische Säuren, wie zum Beispiel Essigsäure oder Milchsäure verwendet werden.

Bei den Zuckern werden Glucose, Fructose, Sacharose, Mischungen aus Glucose und Fruktose, und Mischungen aus Glucose, Fructose und Saccharose bevorzugt. Gegebenenfalls wird Saccharose besonders bevorzugt.

Bei den Alkoholen wird Glycerin bevorzugt.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium-haltigen Salze verwendet werden.

Das Kulturmedium muß weiterhin Salze beispielsweise in Form von Chloriden oder Sulfaten von Metallen wie beispielsweise Natrium, Kalium, Magnesium, Calcium und Eisen enthalten, wie zum Beispiel Magnesiumsulfat oder Eisensulfat, die für das Wachstum notwendig sind. Schließlich können essentielle Wuchsstoffe wie Aminosäuren beispielsweise Homoserin und Vitamine beispielsweise Thiamin, Biotin oder Pantothensäure zusätzlich zu den oben genannten Stoffen eingesetzt werden.

Die genannten Einsatzstoffe können zur Kultur in Form eines einmaligen Ansatzes hinzugegeben oder in geeigneter Weise während der Kultivierung zugefüttert werden.

Zur pH-Kontrolle der Kultur werden basische Verbindungen wie Natriumhydroxid, Kaliumhydroxid, Ammoniak beziehungsweise Ammoniakwasser oder saure Verbindungen wie Phosphorsäure oder Schwefelsäure in geeigneter Weise eingesetzt. Der pH wird im Allgemeinen auf einen Wert von 6,0 bis 8,5 vorzugsweise 6,5 bis 8 eingestellt. Zur Kontrolle der Schaumentwicklung können Antischaummittel, wie zum Beispiel Fettsäurepolyglykolester eingesetzt werden. Zur Aufrechterhaltung der Stabilität von Plasmiden können dem Medium geeignete, selektiv wirkende Stoffe, wie zum Beispiel Antibiotika hinzugefügt werden. Die Fermentation wird bevorzugt unter aeroben Bedingungen durchgeführt. Um diese aufrechtzuerhalten, werden Sauerstoff oder Sauerstoff-haltige Gasmischungen, wie zum Beispiel Luft in die Kultur eingetragen. Die Verwendung von Flüssigkeiten, die mit Wasserstoffperoxid angereichert sind, ist ebenfalls möglich. Gegebenenfalls wird die Fermentation bei Überdruck, beispielsweise bei einem Überdruck von 0,03 bis 0,2 MPa, gefahren. Die Temperatur der Kultur liegt normalerweise bei 20°C bis 45°C und vorzugsweise bei 25°C bis 40°C, besonders bevorzugt bei 30° bis 37°C. Bei batch-Verfahren wird die Kultivierung bevorzugt solange fortgesetzt, bis sich eine für die Maßnahme der Gewinnung des gewünschten L-Ornithins ausreichende Menge, gebildet hat. Dieses Ziel wird normalerweise innerhalb von 10 Stunden bis 160 Stunden erreicht. Bei kontinuierlichen Verfahren sind längere Kultivierungszeiten möglich. Durch die Tätigkeit der Bakterien kommt es zu einer Anreicherung bzw. Zunahme der Konzentration (Akkumulation) des L-Ornithins im Fermentationsmedium.

Beispiele für geeignete Fermentationsmedien finden sich unter anderem in den Patentschriften JP 43010996 B4 (für B. subtilis), US 3668072 A (für E.coli) und JP 57041912 B (für B. flavum).

Gegebenenfalls beträgt das Volumen des Fermentationsmediums in einem erfindungsgemäßen Verfahren ≥ 0,5 l, ≥ 1 l, ≥ 5 l, ≥ 10 l, ≥ 50 l, ≥ 100 l, ≥ 500 l, ≥ 1000 l, bevorzugt ≥ 1 l, besonders bevorzugt ≥ 10 l, ganz besonders bevorzugt ≥ 100 l und äußerst bevorzugt ≥ 1000 l.

Die Analyse von L-Ornithin zur Bestimmung der Konzentration zu einem oder mehreren Zeitpunkt(en) im Verlauf der Fermentation kann durch Trennung der L-Aminosäuren mittels Ionenaustauschchromatographie vorzugsweise Kationenaustauschchromatographie mit anschließender Nachsäulenderivatisierung unter Verwendung von Ninhydrin erfolgen, so wie bei Spackman et al. (Analytical Chemistry 30: 1190-1206 (1958)) beschrieben. Anstelle von Ninhydrin kann auch ortho-Phtadialdehyd zur Nachsäulenderivatisierung eingesetzt werden. Einen Übersichtsartikel zur Ionenaustauschchromatographie findet man bei Pickering (LC·GC (Magazine of Chromatographic Science) 7(6), 484-487 (1989)).

Es ist ebenfalls möglich eine Vorsäulenderivatisierung beispielsweise unter Verwendung von ortho-Phtadialdehyd oder Phenylisothiocyanat vorzunehmen und die entstandenen Aminosäurederivate durch Reversed-Phase-Chromatographie (RP) vorzugsweise in Form der Hochleistungsflüssigkeitschromatographie (HPLC) aufzutrennen. Eine derartige Methode ist beispielsweise bei Lindroth et al. (Analytical Chemistry 51: 1167-1174 (1979)) beschrieben. Die Detektion erfolgt photometrisch (Absorption, Fluoreszenz).

Eine zusammenfassende Darstellung zur Aminosäureanalyse findet man unter anderem im Lehrbuch "Bioanalytik" von Lottspeich und Zorbas (Spektrum Akademischer Verlag, Heidelberg, Deutschland 1998).

Die Leistung der erfindungsgemäßen Verfahren bzw. Fermentationsprozesse bezüglich eines oder mehrerer der Parameter ausgewählt aus der Gruppe der L-Ornithin-Konzentration (gebildetes L-Ornithin pro Volumen), der L-Ornithin-Ausbeute (gebildetes L-Ornithin pro verbrauchter Kohlenstoff-Quelle), der L-Ornithin-Bildung (gebildetes L-Ornithin pro Volumen und Zeit) und der spezifischen L-Ornithin-Bildung (gebildetes L-Ornithin pro Zelltrockenmasse bzw. Biotrockenmasse und Zeit oder gebildetes L-Ornithin pro Zellprotein und Zeit) oder auch anderer Prozess-Parameter und Kombinationen davon, wird um mindestens 0,5%, mindestens 1%, mindestens 1,5% oder mindestens 2% erhöht, bezogen auf Verfahren bzw. Fermentationsprozesse mit Bakterien, in denen ein Protein mit L-Ornithin-Export-Aktivität nicht überexprimiert vorliegt bzw. bei denen keine Maßnahme der Überexpression durchgeführt wurde.

Durch die Maßnahmen der Fermentation erhält man eine Fermentationsbrühe, welche das gewünschte L-Ornithin enthält.

Anschließend erfolgt die Bereitstellung bzw. Herstellung oder Gewinnung eines L-Ornithin haltigen Produktes in flüssiger oder fester Form.

Unter einer Fermentationsbrühe versteht man ein Fermentationsmedium bzw. Nährmedium, in dem ein Mikroorganismus für eine gewisse Zeit und bei einer gewissen Temperatur kultiviert wurde. Das Fermentationsmedium beziehungsweise die während der Fermentation eingesetzten Medien enthält/enthalten sämtliche Substanzen beziehungsweise Komponenten, die eine Produktion des L-Ornithins und typischerweise die Vermehrung bzw. Lebensfähigkeit sicherstellen.

Bei Abschluss der Fermentation enthält die entstandene Fermentationsbrühe dementsprechend
a) die infolge der Vermehrung der Zellen des Bakteriums entstandene Biomasse (Zellmasse) des Bakteriums,
b) das im Laufe der Fermentation gebildete L-Ornithin,
c) die im Laufe der Fermentation gebildeten organischen Nebenprodukte, und
d) die durch die Fermentation nicht verbrauchten Bestandteile des eingesetzten Fermentationsmediums beziehungsweise der Einsatzstoffe wie beispielsweise Vitamine wie Biotin oder Salze wie Magnesiumsulfat.

Zu den organischen Nebenprodukten gehören Stoffe, die von den bei der Fermentation eingesetzten Bakterien neben dem L-Ornithin erzeugt und gegebenenfalls ausgeschieden werden. Hierzu gehören auch Zucker wie zum Beispiel Trehalose.

Die Fermentationsbrühe wird dem Kulturgefäß beziehungsweise dem Fermentationsbehälter entnommen, gegebenenfalls gesammelt, und dazu verwendet, ein L-Ornithin-haltiges Produkt in flüssiger oder fester Form bereitzustellen. Hierfür wird auch der Ausdruck "Gewinnen des L-Ornithin haltigen Produktes" verwendet. Im einfachsten Fall stellt die dem Fermentationsbehälter entnommene L-Ornithin-haltige Fermentationsbrühe selbst das gewonnene Produkt dar.

Durch eine oder mehrere der Maßnahmen ausgewählt aus der Gruppe
a) teilweise (> 0% bis < 80%) bis vollständige (100%) oder nahezu vollständige (≥ 80%, ≥ 90%, ≥ 95%, ≥ 96%, ≥ 97%, ≥ 98% oder ≥ 99% bis < 100%) Entfernung des Wassers,
b) teilweise (> 0% bis < 80%) bis vollständige (100%) oder nahezu vollständige (≥ 80%, ≥ 90%, ≥ 95%, ≥ 96%, ≥ 97%, ≥ 98% oder ≥ 99% bis < 100%) Entfernung der Biomasse, wobei diese gegebenenfalls vor der Entfernung inaktiviert wird,
c) teilweise (> 0% bis < 80%) bis vollständige (100%) oder nahezu vollständige (≥ 80%, ≥ 90%, ≥ 95%, ≥ 96%, ≥ 97%, ≥ 98%, ≥ 99%, ≥ 99,3% oder ≥ 99,7% bis < 100%) Entfernung der im Laufe der Fermentation gebildeten organischen Nebenprodukte, und
d) teilweise (> 0%) bis vollständige (100%) oder nahezu vollständige (≥ 80%, ≥ 90%, ≥ 95%, ≥ 96%, ≥ 97%, ≥ 98%, ≥ 99%, ≥ 99,3% oder ≥ 99,7% bis < 100%) Entfernung der durch die Fermentation nicht verbrauchten Bestandteile des eingesetzten Fermentationsmediums beziehungsweise der Einsatzstoffe,
aus der Fermentationsbrühe erzielt man eine Konzentrierung bzw. Reinigung des L-Ornithins. Auf diese Weise werden Produkte isoliert, die einen gewünschten Gehalt an L-Ornithin aufweisen.

Die teilweise (> 0% bis < 80%) bis vollständige (100%) oder nahezu vollständige (≥ 80% bis < 100%) Entfernung des Wassers (Maßnahme a)) wird auch als Trocknung bezeichnet.

In einer Variante des Verfahrens gelangt man durch vollständige oder nahezu vollständige Entfernung des Wassers, der Biomasse, der organischen Nebenprodukte und der nicht verbrauchten Bestandteile des eingesetzten Fermentationsmediums zu reinen ((≥ 80 Gew.-% oder ≥ 90 Gew.-%) oder hochreinen (≥ 95 Gew.-%, ≥ 97 Gew.-% oder ≥ 99% Gew.-%) Produktformen des L-Ornithins. Für die Maßnahmen gemäß a), b), c) oder d) sind im Stand der Technik eine Fülle von technischen Anleitungen verfügbar. Im Falle der Aminosäure L-Ornithin bzw. deren Salze sind im Stand der Technik im Wesentlichen drei verschiedene Produkte beschrieben.

Eine Gruppe beschreibt das L-Ornithin HCL, bei welchem das L-Ornithin aus der Fermentationslösung nach Zellabtrennung mittels Ionentauscher aufgereinigt wird und dann über Kristallisation als L-Ornithin Monochlorid und Rekristallisation als L-Ornithin Monochlorid kristallisiert wird (US 2988489). Das erhaltene L-Ornithin-HCL hat dabei eine Reinheit größer als > 90%, beispielsweise größer als 95%, beispielsweise größer als 98,0% und beispielsweise größer als 99%. Ein weiteres Verfahren ist beschrieben in der Patentanmeldung EP 1995322. Hierbei wird die biomassehaltige Fermentationsloesung von oben auf ein schwach sauren Ionentauscher mit einer Partikeldurchmesser > 300µm geben und das L-Ornithin über diesen Schritt aufgereinigt. Durch die Auswahl eines entsprechenden Partikeldurchmesser wird eine Verblockung des Harzes durch die Biomasse verhindert. Die Effizienz der Zellabtrennung lag bei 99%.

Das aufgereinigte L-Ornithin kann dann zur Herstellung von verschiedenen L-Ornithin Salzen wie z.B. Mono- oder Di-L-Ornithin-α-Ketoglutarat, L-Ornithin-L-Aspartat etc. eingesetzt werden.

Ein Verfahren zur Herstellung von L-Ornithin-L-Aspartat ist z.B. im Patent EP 0477 991 beschrieben. Dabei wird zu einer wässrigen Lösung aus L-Ornithin und L-Asparatat ein wasserlösliches Lösungsmittel gegeben, um eine min. 90% gesättigte oder übersättigte Lösung zu bekommen. Diese wird unter Rückfluss erhitzt, bis die Bildung von Kristallen beendet ist. Dann wird weiter ein wassermischbares Lösungsmittel unter Rückfluss zugegeben, bis sich die Salzkristalle bilden. Die Kristalle können z.B. über Zentrifugation abgetrennt werden und werden im Anschluss unter Vakuum getrocknet. Die Produktreinheit liegt typischer Weise über 98,5%.

Im Patent JP46003194 ist ein Prozess zur Herstellung von L-Ornithin-L-Ketoglutarat beschrieben. Dabei wird z.B. Ornithin-HCL durch Adsorption an einen sauren Ionentauscher und Elution mit Ammoniakwasser in die freie Base überführt, α-Ketoglutarat zugeben und die Lösung unter Vakuum eingedampft bis das Produkt kristallisiert.

Das Plasmid pEC7lysE wurde in Form des Stammes Escherichia coli DH5alpha/pEC7lysE(DM2204) am 15. Januar 2010 bei der Deutschen Sammlung von Mikroorganismen und Zellkulturen (DSMZ, Braunschweig, Deutschland) unter der Zugangsnummer DSM 23239 nach dem Budapester Vertrag hinterlegt.

### Beispiele

### Beispiel 1:

### Klonierung und Sequenzierung des lysE - Gens aus Corynebacterium glutamicum ATCC 13032

Das Gen lysE des Stammes ATCC13032 wurde in den E. coli / C. glutamicum Shuttle- und Expressionsvektor pVWEx1 (Peters-Wendisch et al., J. Mol. Microbiol. Biotechnol. (2001) 3(2) : 295-300) kloniert.

Die Klonierung wurde in zwei Schritten durchgeführt. Zunächst wurde durch eine Polymerasekettenreaktion (PCR) das Gen aus Corynebacterium glutamicum ATCC13032 mittels folgender, aus Seq ID NO:1 abgeleiteter Oligonukleotid-Primer amplifiziert. Die Oligonukleotide enthielten an ihrem 5'-Ende zusätzliche Restriktionsschnittstellen (unterstrichen: EcoRV für lysE_1.p und AvrII bzw. SspI für lysE_2 .p).
lysE_1.p: 5'-[TCGATATCATGGAAATCTTCATTACAGG]-3' (siehe SEQ ID NO:22)
lysE_2.p: 5'-[TGCCTAGGTCAATATTTGGGCGAAGGCCACCG]-3' (siehe SEQ ID NO:23)

Die PCR-Reaktion wurde in Gegenwart von 200 µM Desoxynukleosid-triphosphaten (dATP, dCTP, dGTP, dTTP), je 0,5 µM des entsprechenden Oligonukleotids, 100 ng chromosomaler DNA von Corynebacterium glutamicum ATCC13032, 1/5 Volumen 5-fach Reaktionspuffer HF und 0,02 U/µl Phusion® Hot Start DNA Polymerase (Biozym Scientific GmbH, D-31840 Hess. Oldendorf) in einem Thermocycler (Mastercycler, Eppendorf AG, Hamburg) unter folgenden Bedingungen durchgeführt: 98°C für 1 min; 30 Zyklen x (98°C, 20 s; 63°C, 20s; 72°C, 40s); 72°C für 6 min.

Das 761 bp grosse PCR-lysE-Fragment (siehe SEQ ID NO:3) wurde, wie im Folgenden beschrieben, in pVWEx1 kloniert: Vorbereitung des Vektors: 1 µg Plasmid-DNA von pVWEx1 wurde im enzymspezifischen Puffersystem mit 10 Units des Enzyms PstI durch Inkubation bei 37°C für 1 h gespalten. Direkt im Anschluss wurde der Spaltungsansatz mit dem Quick Blunting Kit (New England Biolabs GmbH, Frankfurt am Main) nach Herstellerangaben behandelt und danach durch Verwendung des QiaExII-Aufreinigungskits (Qiagen AG, Hilden)nach Herstellerangaben aufgereinigt. Der so vorbehandelte Vektor wurde dann mit 10 Units XbaI im enzymspezifischen Puffersystem bei 37°C für 1 h gespalten und danach erneut mit dem QiaExII-Aufreinigungskit aufgereinigt.

Vorbereitung des Inserts: Das lysE-PCR-Fragment wurde mit jeweils 10 Units Enzym AvrII und EcoRV gespalten und danach durch Verwendung des QiaExII-Aufreinigungskits nach Herstellerangaben aufgereinigt.

Ligation: Vektor und Insert wurden in einem molaren Verhältnis von 1:5 gemischt und durch Verwendung von T4-DNA-Ligase bei 16°C für 1 h ligiert. Vom Ligationsansatz wurden 3 µl in chemisch kompetente E.coli DH5alpha Zellen (Subcloning efficieny, Invitrogen GmbH, Karlsruhe) transformiert.

Transformanten wurden anhand ihrer Kanamycin-Resistenz auf 50 µg/ml Kanamycin-Sulfat enthaltenen LB-Agarplatten identifiziert. Aus 4 der Transformanten wurde Plasmid-DNA isoliert und die Plasmide durch Restriktionsanalyse auf Vorhandensein des 0,75 kb-Fragments als Insert überprüft. Das so entstandene rekombinante Plasmid wurde pVWEx1_lysE bezeichnet.

Die Nukleotidsequenz des 0,75 kb Fragments in Plasmid pVWEx1-lysE wurde nach der Dideoxy-Kettenabbruchmethode nach Sanger et al. bestimmt (Proceedings of the National Academy of Sciences of the United States of America (1977) 74: 5463-5467). Hierzu wurde das vollständige Insert des Plasmides pVWEx1_lysE mit Hilfe der Oligonukleotid-Primer pVW_1.p (5'-TGA GCG GAT AAC AAT TTC ACA C-3')und pVW_2.p(5'-CGA CGG CCA GTG AAT TCG AG-3') bei der Firma Eurofins MWG Operon GmbH (Ebersberg, Deutschland) sequenziert.

Die erhaltene Nukleotidsequenz wurde mit dem Programm Clone Manager 9 analysiert und ist als SEQ ID NO:20. wiedergegeben.

### Beispiel 2:

### Konstruktion des Vektors pK18mobsacB_DargFRGH zur Deletion des argFRGH-Bereiches in Corynebacterium glutamicum

Hierzu wurde zuerst chromosomale DNA aus C. glutamicum ATCC13032 nach der Methode von Tauch et al. (1995, Plasmid 33:168-179) isoliert. Auf der Basis der Sequenz der Gene argFRGH aus C. glutamicum, wurden die unten aufgeführten Oligonukleotide zur Herstellung des argFRGH-Deletionskonstruktes ausgewählt. Das Deletionskontrukt wurde mit Hilfe der Polymerase Kettenreaktion (PCR), im speziellen durch die gene SOEing Methode (Gene Splicing by Overlap Extension, Horton, Molecular Biotechnology 3: 93-98 (1995)) generiert.
argFRGH_d1:
   5'-GGT GGT GCT AGC CCG GCG ATT TCT TTG CAC AT- 3' (siehe SEQ ID NO:24)
argFRGH_d2:
   5'-AAT GCT TAT CGA CGT ACC CCC CTG TGG TTG TGA AGT CAT A-3' (siehe SEQ ID NO:25)
argFRGH_d3:
   5' - GGG GTA CGT CGA TAA GCA TT-3'
   (siehe SEQ ID NO:26)
argFRGH_d4:
   5'-GGT GGT ATG CAT GGT GAT GGT TCC GAA TGT TG-3'
   (siehe SEQ ID NO:27)

Die dargestellten Oligonukleotid-Primer wurden von Eurofins MWG Operon GmbH (Ebersberg, Deutschland) bezogen. Die PCR Reaktion wurde unter Verwendung der Phusion® Hot Start DNA Polymerase (Biozym Scientific GmbH, D-31840 Hess. Oldendorf) in einem Thermocycler (Mastercycler, Eppendorf AG, Hamburg) durchgeführt.

Der Primer argFRGH_d2 ist zusammengesetzt aus zwei Bereichen. Ein Teil der Nukleotidabfolge ist komplementär zum Bereich von 1 bp stromaufwärts bis 19 bp stromabwärts des Startcodons des argF-Gens. Der andere Teil der Nukleotidabfolge ist komplementär zum Bereich von Nukleotid 1419 des argH-Gens bis 5 Nukleotide stromabwärts des argH-Gens.

Mit Hilfe der Polymerase-Kettenreaktion ermöglichen die Primer argFRGH_1 und argFRGH _2 die Amplifikation eines 543 bp großen DNA-Fragmentes und die Primer argFRGH _3 und argFRGH_4 die Amplifikation eines 513 bp großen DNA-Fragmentes. Die Amplifikate wurden per PCR hergestellt, in einem 0,8%igen Agarosegel elektrophoretisch geprüft, mit dem High Pure PCR Product Purification Kit (Product No. 1732676, Roche Diagnostics GmbH, Mannheim, Deutschland) aus dem Agarosegel isoliert und zusammen als Template für eine weitere PCR-Reaktion mit den Primern argFRGH _1 und argFRGH _4 eingesetzt. Auf diese Weise wurde das 1036 bp große DargFRGH-Deletionsderivat erzeugt (siehe auch SEQ ID NO: 21). Es beeinhaltet 477 bp des 3'-Endes des argD-Gens, 19 bp des 5'-Endes des argF-Gens, 15 bp des 3'-Endes des argH-Gens sowie 420 bp des 5'-Endes des cg1589-Leserasters. Das so amplifizierte Produkt wurde in einem 0,8%igen Agarosegel elektrophoretisch geprüft.

Das 1,04 kb große DargFRGH PCR-Produkt (SEQ ID NO: 21) wurde vollständig mit den Enzymen NdeI und NsiI gespalten. Anschließend wurde das Fragment mit dem PCR-Purification Kit (Qiagen, Hilden) aufgereinigt. Das auf diese Weise vorbehandelete DargFRGH-Deletionsderivat wurde mit dem mobilisierbaren Klonierungsvektor pK18mobsacB (Schäfer et al. (1994), Gene 14: 69-73) zur Ligation eingesetzt. Dieser war zuvor mit den Restriktionsendonuklease XbaI und PstI vollständig gespalten worden. Dadurch wurden kompatible DNA-Enden zu den mit NdeI- und NsiI-Spaltung erzeugten Enden des Inserts geschaffen. Der so vorbereitete Vektor wurde mit dem DargFRGH-Fragment in einem molaren Verhältnis von 1:5 gemischt und durch Verwendung von T4-DNA-Ligase (Amersham- Pharmacia, Freiburg, Germany) bei 16°C für 1 Stunde ligiert. Vom Ligationsansatz wurden 3 µl in chemisch kompetente E.coli DH5alpha Zellen (Subcloning efficieny, Invitrogen GmbH, Karlsruhe) transformiert. Transformanten wurden anhand ihrer Kanamycin-Resistenz auf 50 µg/ml Kanamycin-Sulfat enthaltenen LB-Agarplatten identifiziert. Aus 4 der Transformanten wurde Plasmid-DNA isoliert (QIAprep Spin Miniprep Kit der Firma Qiagen (Hilden)) und die Plasmide durch Restriktionsanalyse auf Vorhandensein des 1,04 kb-Fragments als Insert überprüft. Das so entstandene rekombinante Plasmid wurde pK18mobsacB_DargFRGH bezeichnet. Der Stamm wurde als E.coli_DH5alpha/pK18mobsacB_DargFRGH bezeichnet.

Die Nukleotidsequenz des 1,04 kb Fragments (SEQ ID NO:21) in Plasmid pK18mobsacB_DargFRGH wurde nach der Dideoxy-Kettenabbruchmethode nach Sanger et al. durchgeführt (Proceedings of the National Academy of Sciences of the United States of America (1977) 74: 5463-5467). Hierzu wurde das vollständige Insert des Plasmides pK18mobsacB_DargFRGH mit Hilfe der Oligonukleotid-Primer M13 uni (-21) (5'-TGT AAA ACG ACG GCC AGT-3') und M13 rev (-49)(5'- GAG CGG ATA ACA ATT TCA CAC AGG-3') bei der Firma Eurofins MWG Operon (Ebersberg, Deutschland) sequenziert und so auf Richtigkeit überprüft.

### Beispiel 3:

### Herstellung des Stammes Corynebacterium glutamicum ATCC 13032_DargFRGH

Der in Beispiel 2 genannte Vektor pK18mobsacB_DargFRGH wurde mittels Konjugation nach einem Protokoll von Schäfer et al. (Journal of Microbiology 172: 1663-1666 (1990)) in den Corynebacterium glutamicum Stamm ATCC13032 transferiert. Dazu wurde der Vektor zuvor in Stamm E.coli S17-1 transformiert (Simon et al., Biotechnolgy 1:784-791). Die Identität des Vektors in S17-1 wurde Analog zum Nachweis in E.coli DH5alpha (siehe Beispiel 2) geprüft.

Der Vektor pK18mobsacB bzw. pK18mobsacB_DargFRGH kann in C. glutamicum ATCC13032 nicht selbständig replizieren und bleibt nur dann in der Zelle erhalten, wenn er als Folge eines Rekombinationsereignisses ins Chromosom integriert hat. Die Selektion von Klonen mit integriertem pK18mobsacB_DargFRGH erfolgt durch Ausplattieren des Konjugationsansatzes auf LB-Agar (Sambrook et al., Molecular Cloning: A Laboratory Manual, 2nd Ed., Cold Spring Habor, New York, 1989), der mit 15 mg/l Kanamycin und 50 mg/ml Nalidixinsäure supplementiert worden ist. Angewachsene Klone werden auf LB-Agarplatten mit 25 mg/l Kanamycin ausgestrichen und für 16 Stunden bei 33°C inkubiert. Zur Selektion von Mutanten, bei denen als Folge eines zweiten Rekombinationsereignisses die Exzision des Plasmides stattgefunden hat, werden die Klone 20 Stunden unselektiv in LB-Flüssigmedium kultiviert, anschließend auf LB-Agar mit 10% Sucrose ausgestrichen und 24 Stunden bebrütet.

Das Plasmid pK18mobsacB_DargFRGH enthält ebenso wie das Ausgangsplasmid pK18mobsacB neben dem Kanamycin-Resistenzgen eine Kopie des für die Levan-Sucrase aus Bacillus subtilis kodierenden sacB-Gens. Die durch Sucrose induzierbare Expression führt zur Bildung der Levan-Sucrase, die die Synthese des für C. glutamicum toxischen Produktes Levan katalysiert. Auf LB-Agar mit Sucrose wachsen daher nur solche Klone an, bei denen das integrierte pK18mobsacB_DargFRGH wiederum exzisiert hat. Bei der Exzision kann zusammen mit dem Plasmid entweder die vollständige chromosomale Kopie von argFRGH exzisieren, oder die unvollständige Kopie mit der internen Deletion von argFRGH.

Ungefähr 40 bis 50 Kolonien wurden auf den Phänotyp "Wachstum in Gegenwart von Sucrose" und "Nicht-Wachstum in Gegenwart von Kanamycin" geprüft. Um nachzuweisen, dass das deletierte argFRGH-Allel im Chromosom verblieben ist, wurden ungefähr 20 Kolonien, die den Phänotyp "Wachstum in Gegenwart von Sucrose" und "Nicht-Wachstum in Gegenwart von Kanamycin" aufweisen, nach der Standard-PCR-Methode von Innis et al. (PCR Protocols. A Guide to Methods and Applications, 1990, Academic Press) mit Hilfe der Polymerase-Kettenreaktion untersucht. Hierbei wurde aus der chromosomalen DNA der Kolonien ein DNA-Fragment amplifiziert, welches die umgebendem Bereiche des deletierten argFRGH-Bereiches trägt. Es wurden die folgenden Primer-Oligonukleotide für die PCR ausgewählt.
argFRGH_d1 (SEQ ID NO:24):
   5'-GGT GGT GCT AGC CCG GCG ATT TCT TTG CAC AT-3'
argFRGH_d4 (SEQ ID NO:27):
   5'-GGT GGT ATG CAT GGT GAT GGT TCC GAA TGT TG-3'

Die Primer ermöglichen bei Kontroll-Klonen mit vollständigem argFRGH-Lokus die Amplifizierung eines ca. 5,35 kb großen DNA-Fragmentes. Bei Klonen mit einem deletierten argFRGH-Lokus werden DNA-Fragmente mit einer Größe von ca. 1,04 kb amplifiziert.

Die amplifizierten DNA-Fragmente wurden mittels Elektrophorese in einem 0,8%igen Agarosegel identifiziert. Damit konnte gezeigt werden, daß der Stamm ein deletiertes argFRGH-Allel auf dem Chromosom trägt. Der Stamm wurde als Corynebacterium glutamicum Delta_argFRGH bezeichnet.

### Beispiel 4:

### Expression des lysE-Gens in Corynebacterium glutamicum ATCC 13032_Delta_argFRGH

Mittels Elektroporation (Haynes et al., FEMS Microbiology Letters (1989) 61: 329-334) wurde das Plasmid pVWEx1_LysE und das Leerplasmid pVWEx1 in den L-Ornithin bildenden Stamm ATCC 13032_Delta_argFGH eingebracht. Transformanten wurden anhand ihrer Kanamycinresistenz auf 25 µg/ml Kanamycin enthaltenen Caso-Agarplatten identifiziert. 5 Einzelklone wurden im Anschluss auf Korrektheit des transformierten Plasmides geprüft. Dazu wurde Plasmid-DNA isoliert (Plasmid Isolation Kit, Qiagen) und diese DNA durch Restriktionsanalyse auf das korrekte Spaltungsmuster überprüft. Auf diese Weise entstanden die C. glutamicum Stämme ATCC 13032_Delta_argFRGH/pVWEx1_lysE und ATCC 13032_Delta_argFRGH/pVWEx1.

### Beispiel 5:

### Herstellung von L-Ornithin mit Corynebacterium glutamicum

Zur Untersuchung ihrer Fähigkeit L-Ornithin zu produzieren wurden jeweils drei Klone des Stammes ATCC 13032_Delta_argFRGH/pVWEx1_lysE und drei Klone des Stammes ATCC 13032_Delta_argFRGH/pVWEx1 in jeweils 10 ml Testmedium für 16 h bei 33°C vorkultiviert. Für den Produktionstest wurden mit der erhaltenen Vorkultur je 10 ml Testmedium so angeimpft, dass die Start-OD₆₀₀ (optische Dichte bei 600 nm) 0,1 betrug. Jeder Klon wurde in drei Schüttelkolben geprüft, sodass jeder Stamm zum jeweiligen Erntezeitpunkt durch insgesamt neun Schüttelkolben repräsentiert ist. Das Testmedium war identisch mit dem bei Keilhauer et al. (Journal of Bacteriology (1993) 175: 5593-5603) beschriebenen CgXII-Medium, enthielt aber zusätzlich 7,5 g/l Hefeextract (Difco), 25 µg/ml Kanamycin, 1 mM IPTG (isopropyl-beta-D-thiogalactopyranoside) und 40 g/l Saccharose anstelle Glucose. Der Einfachheit halber ist die Zusammensetzung des Testmediums in der nachfolgenden Tabelle 2 zusammengefasst.

**Tabelle 2**

| **Komponente** | **Gehalt pro l** |
|---|---|
| (NH₄)₂SO₄ | 20 g |
| Harnstoff | 5 g |
| KH₂PO₄ | 1 g |
| K₂HPO₄ | 1 g |
| MgSO₄ x 7 H₂O | 0,25 g |
| 3-Morpholinopropansulfonsäure (MOPS) | 42 g |
| CaCl₂ | 0,01 g |
| FeSO₄ x 7H₂O | 0,01 g |
| MnSO₄ x H₂O | 0,01 g |
| ZnSO₄ x 7 H₂O | 0,001 g |
| CuSO₄ | 0,0002 g |
| NiCl₂ x 6H₂O | 0,00002 g |
| Biotin | 0,0002 g |
| Protokatechusäure | 0,03 g |
| Saccharose | 40 g |
| Hefeextrakt | 7,5 g |
| pH (mit NaOH) | 7 |

Die Kultivierung erfolgte bei 33°C und 200 rpm in 100 ml Schüttelkolben. Die Auslenkung des Schüttlers war 5 cm. Nach 24 und 48 Stunden wurden drei Kulturen eines Klons geerntet. Hierzu wurden Proben aus den Kulturen entnommen und die optische Dichte, der Gehalt an Saccharose und der Gehalt an L-Ornithin bestimmt. Für die Bestimmung Gehaltes an Saccharose und L-Ornithin wurden die Zellen kurz abzentrifugiert (Tischzentrifuge Typ 5415D (Eppendorf) bei 13000 rpm, 10 min, Raumtemperatur).

Die Bestimmung der optischen Dichte erfolgte bei einer Wellenlänge von 660 nm mit einem GENios Mikrotiterplatten Photometer (Tecan, Reading UK). Die Proben wurden vor der Messung 1:100 mit demineralisiertem Wasser verdünnt.

Die Bestimmung der Saccharose erfolgte mit einem Testsystem (Cat. Nr. 10 716 251 035) der Firma R-Biopharm AG (Darmstadt, Deutschland). Hierbei wird Saccharose invertiert und die gebildete Glukose mit einem gekoppeltem Enzymtest (Hexokinase/Glukose-6-Phosphat Dehydrogenase) via NADH-Bildung nachgewiesen.

Die quantitative Bestimmung der extrazellulären Aminosäurekonzentrationen aus dem Kulturüberstand erfolgte mittels reversed phase HPLC (Lindroth et al., Analytical chemistry (1979) 51: 1167-1174). Es wurde ein HPLC-Gerät der Serie HP1100 (Hewlett-Packard, Waldbronn, Deutschland) mit angeschlossenem Fluoreszenzdetektor (G1321A) verwendet; die Systemsteuerung und die Auswertung der Daten erfolgte mit einer HP-Chem-Station (Hewlett-Packard). 1 µL der zu analysierenden Aminosäurelösung wurde in einer automatischen Vorsäulenderivatisierung mit 20 µL ortho-Phthalaldehyd/2-Mercaptoethanol-Fertigreagenz (Pierce Europe BV, Oud-Beijerland, Niederlande) gemischt. Die dabei entstehenden fluoreszierenden, thiosubstituierten Isoindole (Jones et al., Journal of Chromatography (1983) 266: 471-482) wurden über eine kombinierte Vorsäule (40x4 mm Hypersil ODS 5) und Hauptsäule (Hypersil ODS 5, beide Säulen von der Firma CS-Chromatographie Service GmbH, Langerwehe, Deutschland) mit einem Gradientenprogramm mit zunehmend unpolarer Phase (Methanol) aufgetrennt. Das polare Eluenz war Natriumacetat (0,1 M; pH 7,2); die Flußrate betrug 0,8 mL pro Minute. Die Fluoreszenzdetektion der derivatisierten Aminosäuren erfolgte bei einer Anregungswellenlänge von 230 nm und einer Emissionswellenlänge von 450 nm. Die L-Ornithin bzw. die L-Ornithinhydrochlorid Konzentrationen wurden durch einen Vergleich mit einem externen Standard und L-Asparagin als zusätzlichen internen Standard berechnet.

Das Molekulargewicht von L-Ornithinhydrochlorid beträgt 168,6 g x mol⁻¹ und von L-Ornithin 132,1 g x mol⁻¹.

Zur Berechnung der Ausbeute wurde die Menge an gebildetem L-Ornithin (gemessen als L-Ornithinhydrochlorid) durch die Menge an verbrauchter Saccharose geteilt.

Die Ergebnisse sind in Tabelle 3 dargestellt.

Tabelle 3: L-Ornithin Bildung nach 24 Stunden (Tabelle 3A) und 48 Stunden (Tabelle 3B) Inkubation. Abkürzungen: *: ATCC 13032 _Delta_argFRGH; Orn-HCl: L-Ornithinhydrochlorid.

**Tabelle 3A:**

| Zeit | 24 Stunden | | |
|---|---|---|---|
| Stamm | Orn-HCl g/l | Ausbeute g/g | OD |
| */pVWEx1 | 9,83 ± 0,10 | 0,39 ± 0,01 | 10,78 ± 0,30 |
| */pVWEx1_lysE | 13,03 ± 0,16 | 0,44 ± 0,01 | 10,40 ± 0,41 |

**Tabelle 3B:**

| Zeit | 48 Stunden | | |
|---|---|---|---|
| Stamm | Orn-HCl g/l | Ausbeute g/g | OD |
| */pVWEx1 | 15,50 ± 0,74 | 0,36 ± 0,01 | 11,69 ± 1,40 |
| */pVWEx1_lysE | 18,48 ± 0,51 | 0,42 ± 0,01 | 9,18 ± 0,48 |

### Beispiel 6: Sequenzierung und Hinterlegung des Plasmides pEC7lysE

Das Plasmid pEC7lysE wurde von Dr. Lothar Eggeling (Forschungszentrum Jülich GmbH, D-52425 Jülich), dem Korrespondenzautor der Veröffentlichung Bellmann et al. (Microbiology (2001) 147,1765-1774), in Form einer wässrigen Lösung zur Verfügung gestellt.

Von der erhaltenen DNA-Lösung wurde ein Aliquot zur Transformation kompetenter Escherichia coli Zellen des Stammes DH5alpha (subcloning efficiency, Genotyp: F-Φ80*lac*ZΔM15 Δ(*lac*ZYA-*arg*F) U169 recA1 *end*A1 hsdR17 (rK-, mK+) *phoA* supE44 λ- thi-1 gyrA96 *rel*A1) der Firma Invitrogen GmbH (Paisley, UK) nach Herstellerangaben eingesetzt. Die Selektion der Transformanten erfolgte auf Luria-Bertani-Agar, der mit 50 µg/ml Kanamycin supplementiert worden war.

Eine Transformante wurde als Escherichia coli DH5alpha/pEC7lysE(DM2204) bezeichnet und am 15. Januar 2010 bei der Deutschen Sammlung von Mikroorganismen und Zellkulturen GmbH (Braunschweig, Deutschland) unter der Hinterlegungsnummer DSM 23239 nach Budapester Vertrag hinterlegt.

Im Rahmen einer Auftragssequenzierung (Walking Service) bei der Firma Eurofins MWG Operon GmbH (Martinsried, Deutschland) wurde das Plasmid pEC7lysE aus Stamm DSM 23239 vollständig sequenziert. Die Sequenz von pEC7lysE ist als SEQ ID NO:29 angegeben.

## Patentansprüche

1. Verfahren zur Herstellung von L-Ornithin, **dadurch gekennzeichnet, dass** man folgende Schritte durchführt
a) Fermentation eines L-Ornithin ausscheidenden Bakteriums, ausgewählt aus der Gruppe Corynebacterium, Bacillus, Streptomyces, Arthrobacter und der Enterobacteriaceae, in dem ein Polynukleotid, das für ein Polypeptid kodiert, welches die Aktivität eines L-Ornithin-Exporters hat und dessen Aminosäuresequenz mit SEQ ID NO:2 identisch ist oder dessen Aminosäuresequenz durch insgesamt maximal 25 Deletionen, Insertionen, Substitutionen und/oder N- bzw. C-terminale Additionen von Aminosäuren aus SEQ ID NO:2 erhalten werden kann, überexprimiert vorliegt, in einem Medium
b) Akkumulation des L-Ornithins in dem Medium, wobei eine Fermentationsbrühe erhalten wird, und
c) wobei für die Überexpression das Plasmid pEC7lysE hinterlegt in DSM23239 ausgeschlossen wird.

2. Das Verfahren nach Anspruch 1, **dadurch gekennzeichnet, dass** im Falle von Corynebacterium glutamicum durch die Überexpression die Höhe der L-Ornithin Exportaktivität um mindestens 10% erhöht vorliegt im Vergleich zu ATCC13032 oder ATCC14067 oder ATCC13869.

3. Das Verfahren nach Anspruch 1 bis 2, **dadurch gekennzeichnet, dass** die Überexpression durch eine oder mehrere der Maßmahmen erzielt wird ausgewählt aus der Gruppe
a) Erhöhung der Kopienzahl,
b) Verwendung eines starken Promotors, und
c) Mutation des Promotors, und
d) Überexpression eines Aktivatorproteins.

4. Das Verfahren nach Anspruch 1 bis 3, **dadurch gekennzeichnet, dass** es sich bei dem Bakterium um Corynebacterium, bevorzugt Corynebacterium glutamicum handelt.

5. Das Verfahren nach Anspruche 1 bis 4, **dadurch gekennzeichnet, dass** man zusätzlich eines oder mehrere der Gene ausgewählt aus der Gruppe
a) odhA-Gen, das für die E1-Untereinheit der alpha-Ketoglutarat-Dehydrogenase (EC 1.2.4.2) kodiert,
b) sucA-Gen, das für die Dihydrolipoamide Succinyltransferase (EC 2.3.1.61) kodiert,
c) dapA-Gen, das für eine Dihydrodipicolinat-Synthase (DapA, EC 4.2.1.52) kodiert,
d) dapB-Gen, das für eine Dihydrodipicolinat-Synthase (DapB, EC 1.3.1.26) kodiert,
e) ddh-Gen, das für eine meso-Diaminopimelat Dehydrogenase (Ddh, EC 1.4.1.16) kodiert,
f) lysA-Gen, das für eine Diaminopimelat-Decarboxylase (LysA, EC 4.1.1.20) kodiert,
g) argR-Gen, das für einen/den Repressor (ArgR) der Biosynthese von L-Arginin kodiert,
h) argF-Gen, das für eine Ornitin Carbamoyl Transferase (ArgF, EC 2.1.3.3) kodiert,
i) argG-Gen, das für eine Argininosuccinat Synthase (ArgG, EC 6.3.4.5) kodiert,
j) argH-Gen, das für eine Argininosuccinat Lyase (ASAL) (ArgH, EC 4.3.2.1) kodiert,
k) lysC-Gen, das für eine Aspartatkinase (LysC, EC 2.7.2.4) kodiert, und
l) asd-Gen, das für eine Aspartatsemialdehyd-Dehydrogense (Asd, EC 1.2.1.11) kodiert,
abschwächt.

6. Das Verfahren nach Anspruch 1 bis 5, **dadurch gekennzeichnet, dass** man zusätzlich eines oder mehrere der Gene ausgewählt aus der Gruppe
a) Glutamat-Dehydrogenase (EC 1.4.1.3) kodiert vom gdh-Gen,
b) Glutamat N-Acetyltransferase (EC 2.3.1.35 und EC 2.3.1.1) kodiert vom argJ-Gen,
c) Acetylglutamatkinase (EC 2.7.2.8) kodiert vom argB-Gen,
d) N-Acetyl-Gamma-Glutamyl-Phosphat-Reduktase (EC 1.2.1.38) kodiert vom argC-Gen,
e) Acetyl-Ornithin Aminotransferase (EC 2.6.1.11), kodiert vom argD-Gen,
f) Glukose spezifische Komponente EIIB (PtsG) (EC 2.7.1.69) des Glukose-Aufnahmesystems kodiert vom ptsG-Gen,
g) Saccharose spezifische Komponente EIIB (PtsS) (EC 2.7.1.69) des Saccharose-Aufnahmesystems kodiert vom ptsS-Gen,
h) Glucose-6-Phosphat 1- Dehydrogenase (EC 1.1.1.49) kodiert vom zwf-Gen,
i) Glucose-6-Phosphat-Isomerase (EC 5.3.1.9) kodiert vom pgi-Gen,
j) Phoshofructokinase (EC 2.7.1.11) kodiert vom pfkA-Gen,
k) Fructose-bisphosphat aldolase (EC 4.1.2.13) kodiert vom fda-Gen,
l) Glyceraldehyd-3-Phosphat Dehydrogenase (EC 1.2.1.59), kodiert vom gap-Gen,
m) Phosphoglycerat Kinase (EC 2.7.2.3) kodiert vom pgk-Gen,
n) Pyruvatkinase (EC 2.7.1.40) kodiert vom pyk-Gen,
o) E1-Untereinheit der Pyruvat-Dehydrogenase (EC 1.2.4.1) kodiert vom aceE-Gen,
p) Phosphoenolpyruvat-Carboxylase (EC 4.1.1.31) kodiert vom ppc-Gen,
q) Pyruvat-Carboxylase (EC 6.4.1.1), kodiert vom pyc-Gen,
r) Aconitase (EC 4.2.1.3) kodiert vom acn-Gen, und
s) Isocitrat-Dehydrogenase (EC 1.1.1.42) kodiert vom icd-Gen
verstärkt.

7. Das Verfahren nach Anspruch 1 bis 6 **dadurch gekennzeichnet, dass** es sich um ein Verfahren ausgewählt aus der Gruppe Satzverfahren, Zulaufverfahren, repetitives Zulaufverfahren und kontinuierliches Verfahren handelt.

8. Das Verfahren nach Anspruch 1 bis 7, **dadurch gekennzeichnet, dass** man aus der L-Ornithin haltigen Fermentationsbrühe L-Ornithin oder ein flüssiges oder festes L-Ornithin-haltiges Produkt gewinnt.
**Abbildung** 1
Alignment der in Tab. 1 aufgeführten Aminosäuresequenzen der LysE und ArgO Proteine. Alignmentlücken (gaps) sind durch "-" gekennzeichnet. Identische Aminosäuren sind durch einen "*" gekennzeichnet.
| | |
|---|---|
| YP_225551.1 | -------------------MEIFITGLLLGASLLLSIGPQNVLVIKQGIKREGLIAVLLV |
| ZP_05749209.1 | -------------------MEIFVTGLLLGASLLLAIGPQNVLVIKQGIKREGITAVIIV |
| NP_939452.1 | -------------------MSIAIAGFLMGLSLIVAIGPQNALIIRQGIKREGLIPILVV |
| ZP_03933958.1 | -------------------MSVLLAGFLLGLSLIVAIGPQNAYIIKMGVKRDHIGAIILA |
| YP_002834652.1 | MRRLEA-------------MSVLLAGFALGLSLIIAIGPQNAYIIKMGIKRDHVGPILLA |
| ZP_03711883.1 | -------------------MSIAVAGFLLGLSLIVAIGPQNALVIRQGVKREGLIVVAI |
| ZP_03922319.1 | -------------------MSIVLAGFFLGLSLIVAVGPQNAMLLKYGIRRDHIGLIIVV |
| ZP_03931790.1 | -------------------MSIVLAGFVLGLSLIVAVGPQNAMLLKYGIRRDHIGLIIVV |
| ZP_03918361.1 | MVPENLSFYLCVLLTHNKYVNVFFAGLLFNLSLILALGPQNALILKYGLRRQAITLVISV |
| YP_002958101.1 | M------------------WTLAGTGLLTGLALIVAIGAQNAFVLRQGVRREHVGAVVLV |
| ZP_05365683.1 | -------------------MSIVLAGFFLGLSLIVAVGPQNAMLLKYGIRRDHIGLIIVV |
| ZP_04835056.1 | -------------------MSIAVAGFLLGLSLIVAIGPQNALVIRQGVKREGLIVVLAI |
| | * * * ** * * |
| | |
|---|---|
| YP_225551.1 | CLISDVFLFIAGTLGVDLLSNAAPIVLDIMRWGGIAYLLWFAVMAAKDAMTNKVEA---- |
| ZP_05749209.1 | CLLSDVVLFTLGTLGVGLISDTAPIILDILRWCGIAYLLWFAVMAARDALRARTEV---- |
| NP_939452.1 | CILSDVILIFGGTAGVGALVDRAPIALVVLKWLGVAYLLYFGFTCFKEAFKRHGQA---- |
| ZP_03933958.1 | CLLSDVILINAGVGGMGVLVEKFPTGLIIMKYLGAAYLIYFGFTCFRDAFKKEQEA---- |
| YP_002834652.1 | CLLSDVILITGGTAGVGVLVERFPTALVVVKYLGAAYLIYFGFTCFRDAFKKQQDA---- |
| ZP_03711883.1 | CILSDIFLIFGGTAGVGVIIEKAPLALVALKWFGAAYLAWFAVSCFRDMV--KPRA---- |
| ZP_03922319.1 | CALSDVILITSGTAGVGYLVEKFPNALQVLKYVGAAYLAYFTFTCFRDAFKTKGEA---- |
| ZP_03931790.1 | CALSDVILITSGTAGVGYLVERFPNALEALKYIGAAYLAFFTFTCFRDAFKTKGEAIDVE |
| ZP_03918361.1 | CALCDITLIALSGVGVGVILQKAPIVLEILRYAGFLYLLWFAYTCFRDAIHPKTLA---- |
| YP_002958101.1 | CMASDAVLILAGTAGVGALVQAVPWLLEVLRWGGALYLLWFAVSSLRAAL-----R---- |
| ZP_05365683.1 | CALSDVILITSGTAGVGYLVEKFPNALQVLKYVGAAYLAYFTFTCFRDALKTKGEA---- |
| ZP_04835056.1 | CMLSDIFLIFGGTAGVGVIIEKAPLALVALKWFGAAYLAWFAVSCFKDMVKPRALD---- |
| | * * * * * * * ** * |
| | |
|---|---|
| YP_225551.1 | ---PQ-IIE-----ETEP---T--VP-----D-DTP-LG----------G-----SAVAT |
| ZP_05749209.1 | ---T--FVE-----HSEP---V--AA-----A-SAS-GG----------G---------V |
| NP_939452.1 | ----L-AVE-----QSEP---V--AY-----E-PVA-DA----------S-----SGVIT |
| ZP_03933958.1 | ----L-VVS-----STPP---S--AP-----N-ETE-LG----------G------ATTV |
| YP_002834652.1 | ----L-VIE-----ETTP---VAQVV-----D-ENS-GN----------A-----GAPGT |
| ZP_03711883.1 | ---LD-SSA-----TDDG---T--SL-----D-DAP-TAAHVSNVDTTSG-----NGGQV |
| ZP 03922319.1 | ---IE-V-E-----STQP---K--AP-----Q-EVASFD----------G-----SQARS |
| ZP 03931790.1 | STSPN-STE-----EVAT---F--DG-----D-GDS-TG----------GVGTEHGSVAT |
| ZP_03918361.1 | ---TE-TVS-----ETKPHEEE--LP-----DVSST-TA----------G-----TTMAT |
| YP_002958101.1 | ---PQGLMA-----EQAP---R--T-------------A----------G-----SVIAT |
| ZP_05365683.1 | ---IE-V-E-----STQP---K--VP-----Q-EVASFD----------G-----SQARN |
| ZP_04835056.1 | ---SS-ATDNGTSLDDAP---T--VAHISNVD-STS-GN----------G-----GQVQT |
| | |
|---|---|
| YP_225551.1 | DTRNRVRVEVS--VDKQ----RVWVK-------PMLMAIVLTWLNPNAYLDAFVFIGGVG |
| ZP_05749209.1 | TTKQRPRLRIT--SGTR----QVWVR-------PMLMAIVLTWLNPNAYLDAFVFIGGVG |
| NP_939452.1 | KTRTKAQPK-----SAQ----RTWVK-------PVLAALAFTWLNPAAYIDVLVMLGGIA |
| ZP_03933958.1 | MTKQRT---------KS----RTWVK-------PVMGAMALTWLNPLAYVDVLVMLGGIA |
| YP_002834652.1 | SVLTKIRPRV-----RS----KSWVK-------PVLGALALTWLNPLAYVDALVMLGSIA |
| ZP_03711883.1 | QTKTRPITTTA--PTRQAHPARPWVK-------PALAALAFTWLNPSAYIDTLVMLGGIA |
| ZP_03922319.1 | TTKTAARVEIK----RS----PSWVK-------PLLTALALTWLNPGAYVDVVVMLGSIA |
| ZP_03931790.1 | ATATQ-RQEIK----RS----PSWVK-------PLLTALALTWLNPGAYVDVLVMLGGIA |
| ZP 03918361.1 | ATVMETATTVK--EKTH----RRTFHIPQEIKGPAVAAFVVSVINPAAWVDLFVVIGSIS |
| YP_002958101.1 | -------------------------------------TLALTWLNPHVYLDTVVLLGSLA |
| ZP_05365683.1 | TTKTATRVEIK----RS----PSWVK-------PLLTALALTWLNPGAYVDVVVMLGSIA |
| ZP_04835056.1 | KTRPITTTAPTRQAHPA----RPWVK-------PALAALAFTWLNPSAYIDTLVMLGGIA |
| | ** * * * |
| | |
|---|---|
| YP_225551.1 | AQYGDTGRWIFAAGAFAASLIWFPLVGFGAAALSRPLSSPKVWRWINVVVAVVMTALAIK |
| ZP_05749209.1 | AQYGETGRWIFAAGAFAASLVWFPLVGYGAAALSRPLSSPRVWRWINIGVAVVLTGLAVK |
| NP_939452.1 | NQIIGPDGRWVFALGALCASLTWFPFIGYTSTRFSTVLSRPAVWRYINIAIGIIMMIMCAR |
| ZP_03933958.1 | QHYGDQ-RWVFAAGAIMASAVWFPTVGYGAFKLSHVLAKPTTWRYVNFAIGCVMMLLTAK |
| YP_002834652.1 | NQYGDQ-RWVFAGGAILASAVWFPSLGFGAYKLSHVLAKPTTWRVVNIVIGCVMLALTAK |
| ZP_03711883.1 | NQHGESGRWVFAAGALMASAVWFPLLGFFSTRFSRVLSRPQAWRVINGVIGCIMVVMCIR |
| ZP_03922319.1 | NQYGESGRWLFAVGAICASFTWFPFIGFGAARFSHVLSRPTVWRWINFGIGVIMIGLTLK |
| ZP_03931790.1 | NQYGDPGRWLFAGGAIAASFTWFPVIGFGAARFSHVLSRPEVWRWINVGIGVIMIGLTLK |
| ZP_03918361.1 | SSYGP-DKWAFLLGTMAASLVWFPAFGYGAAALSRPLSSPKVWRCINTGIGLFMVFMAFR |
| YP_002958101.1 | NQHGPDARWVFAAGAVAASVLWFTALGYGARLLARVLADPKAWRVVDVVIAVVMAVLAVR |
| ZP_05365683.1 | NQYGESGRWLFAVGAICASFTWFPFIGFGAARFSHVLSRPTVWRWINFGIGVIMIGLTLK |
| ZP_04835056.1 | NQHGESGRWVFAAGALMASAVWFPLLGFFSTRFSRVLSRPQAWRVINGVIGCIMVVCIR |
| | * * * * ** ** * * * ** |
| | |
|---|---|
| YP225551.1 | LMLMG---- |
| _ ZP05749209.1 | LILMG---- |
| _ NP939452.1 | LIMH----- |
| _ ZP03933958.1 | LLLH----- |
| _ YP_002834652.1 | LLFL----- |
| ZP03711883.1 | LVMH----- |
| _ ZP03922319.1 | LLLL----- |
| _ ZP03931790.1 | LLLL----- |
| _ ZP03918361.1 | VLFM----- |
| _ YP002958101.1 | LIAGSDVWG |
| _ ZP05365683.1 | LLLL----- |
| _ ZP_04835056.1 | LIMH----- |
**Abbildung** 2
Einstellung des Programmes Clone Manager 9 der Firma Scientific and Educational Software zur Bestimmung der Identität zweier Aminosäuresequenzen durch ein globales Sequenzalignment.

## Claims

1. Process for the preparation of L-ornithine, **characterized in that** the following steps are carried out:
a) fermentation of an L-ornithine-excreting bacterium selected from the group consisting of Corynebacterium, Bacillus, Streptomyces, Arthrobacter and the Enterobacteriaceae, which bacterium harbours an overexpressed polynucleotide coding for a polypeptide which has the activity of an L-ornithine exporter and whose amino acid sequence is identical to SEQ ID NO:2, or whose amino acid sequence may be derived from SEQ ID NO:2 by a maximum of 25 deletions, insertions, substitutions and/or N- or C-terminal additions of amino acids in total, in a medium,
b) accumulation of L-ornithine in the medium, wherein a fermentation broth is obtained, and
c) wherein, for overexpression, plasmid pEC71ysE deposited in DSM23239 is excluded.

2. Process according to Claim 1, **characterized in that**, in the case of Corynebacterium glutamicum, overexpression increases the level of L-ornithine export activity by at least 10%, compared to ATCC13032 or ATCC14067 or ATCC13869.

3. Process according to Claims 1 to 2, **characterized in that** overexpression is achieved by one or more of the measures selected from the group consisting of
a) increasing the copy number,
b) using a strong promoter, and
c) mutating the promoter, and
d) overexpressing an activator protein.

4. Process according to Claims 1 to 3, **characterized in that** the bacterium is Corynebacterium, preferably Corynebacterium glutamicum.

5. Process according to Claims 1 to 4, **characterized in that** additionally one or more of the genes selected from the group consisting of
a) odhA gene coding for the E1 subunit of alpha-ketoglutarate dehydrogenase (EC 1.2.4.2),
b) sucA gene coding for dihydrolipoamide succinyl transferase (EC 2.3.1.61),
c) dapA gene coding for a dihydrodipicolinate synthase (DapA, EC 4.2.1.52),
d) dapB gene coding for a dihydrodipicolinate synthase (DapB, EC 1.3.1.26),
e) ddh gene coding for a meso-diaminopimelate dehydrogenase (Ddh, EC 1.4.1.16),
f) lysA gene coding for a diaminopimelate decarboxylase (LysA, EC 4.1.1.20),
g) argR gene coding for a/the repressor (ArgR) of L-arginine biosynthesis,
h) argF gene coding for an ornithine carbamoyl transferase (ArgF, EC 2.1.3.3),
i) argG gene coding for an argininosuccinate synthase (ArgG, EC 6.3.4.5),
j) argH gene coding for an argininosuccinate lyase (ASAL) (ArgH, EC 4.3.2.1),
k) lysC gene coding for an aspartate kinase (LysC, EC 2.7.2.4), and
l) asd gene coding for an aspartate semialdehyde dehydrogenase (Asd, EC 1.2.1.11),
is/are attenuated.

6. Process according to Claims 1 to 5, **characterized in that** additionally one or more of the genes selected from the group consisting of
a) glutamate dehydrogenase (EC 1.4.1.3) encoded by the gdh gene,
b) glutamate N-acetyltransferase (EC 2.3.1.35 and EC 2.3.1.1) encoded by the argJ gene,
c) acetylglutamatekinase (EC 2.7.2.8) encoded by the argB gene,
d) N-acetyl-gamma-glutamyl-phosphate reductase (EC 1.2.1.38) encoded by the argC gene,
e) acetylornithine aminotransferase (EC 2.6.1.11), encoded by the argD gene,
f) glucose-specific component EIIB (PtsG) (EC 2.7.1.69) of the glucose uptake system, encoded by the ptsG gene,
g) sucrose-specific component EIIB (PtsS) (EC 2.7.1.69) of the sucrose uptake system, encoded by the ptsS gene,
h) glucose-6-phosphate 1-dehydrogenase (EC 1.1.1.49) encoded by the zwf gene,
i) glucose-6-phosphate isomerase (EC 5.3.1.9) encoded by the pgi gene,
j) phosphofructokinase (EC 2.7.1.11) encoded by the pfkA gene,
k) fructose-bisphosphate aldolase (EC 4.1.2.13) encoded by the fda gene,
l) glyceraldehyde-3-phosphate dehydrogenase (EC 1.2.1.59) encoded by the gap gene,
m) phosphoglycerate kinase (EC 2.7.2.3) encoded by the pgk gene,
n) pyruvate kinase (EC 2.7.1.40) encoded by the pyk gene,
o) E1 subunit of pyruvate dehydrogenase (EC 1.2.4.1), encoded by the aceE gene,
p) phosphoenolpyruvate carboxylase (EC 4.1.1.31) encoded by the ppc gene,
q) pyruvate carboxylase (EC 6.4.1.1), encoded by the pyc gene,
r) aconitase (EC 4.2.1.3) encoded by the acn gene, and
s) isocitrate dehydrogenase (EC 1.1.1.42) encoded by the icd gene,
is/are enhanced.

7. Process according to Claims 1 to 6, **characterized in that** it is a process selected from the group consisting of a batch process, fed-batch process, repetitive fed-batch process, and continuous process.

8. Process according to Claims 1 to 7, **characterized in that** L-ornithine or a liquid or solid L-ornithine-containing product is recovered from the L-ornithine-containing fermentation broth.

## Revendications

1. Procédé de préparation de L-ornithine, **caractérisé en ce qu'**on met en œuvre les étapes suivantes :
a) fermentation, dans un milieu, d'une bactérie secrétant la L-ornithine, choisie dans le groupe Corynebacterium, Bacillus, Streptomyces, Arthrobacter et les Enterobacteriaceae, dans laquelle est présent surexprimé un polynucléotide qui code pour un polypeptide qui a l'activité d'un exportateur de L-ornithine et dont la séquence d'acides aminés est identique à la SEQ ID NO: 2 ou dont la séquence d'acides aminés peut être obtenue par en tout au maximum 25 délétions, insertions, substitutions et/ou additions N- ou C-terminales d'acides aminés de la SEQ ID NO: 2,
b) accumulation de la L-ornithine dans le milieu, avec obtention d'un bouillon de fermentation, et
c) le plasmide pEC7lysE, déposé sous le numéro DSM23239, étant exclu pour la surexpression.

2. Procédé selon la revendication 1, **caractérisé en ce que**, dans le cas de Corynebacterium glutamicum, la hauteur de l'activité d'exportation de la L-ornithine est, sous l'effet de la surexpression, augmentée d'au moins 10 % par rapport à ATCC13032 ou ATCC14067 ou ATCC13869.

3. Procédé selon les revendications 1 à 2, **caractérisé en ce que** la surexpression est obtenue par l'une ou plusieurs des mesures choisies dans le groupe
a) augmentation du nombre de copies,
b) utilisation d'un promoteur fort, et
c) mutation du promoteur, et
d) surexpression d'une protéine activatrice.

4. Procédé selon les revendications 1 à 3, **caractérisé en ce que**, pour ce qui concerne la bactérie, il s'agit de Corynebacterium, de préférence Corynebacterium glutamicum.

5. Procédé selon les revendications 1 à 4, **caractérisé en ce qu'**on atténue en outre un ou plusieurs des gènes choisis dans le groupe
a) le gène odhA, qui code pour la sous-unité E1 de l'alpha-cétoglutarate-déshydrogénase (EC 1.2.4.2),
b) le gène sucA, qui code pour la dihydrolipoamide succinyltransférase (EC 2.3.1.61),
c) le gène dapA, qui code pour une dihydrodipicolinate-synthase (DapA, EC 4.2.1.52),
d) le gène dapB, qui code pour une dihydrodipicolinate-synthase (DapB, EC 1.3.1.26),
e) le gène ddh, qui code pour une méso-diaminopimélate déshydrogénase (Ddh, EC 1.4.1.16),
f) le gène lysA, qui code pour une diaminopimélate-décarboxylase (LysA, EC 4.1.1.20),
g) le gène argR, qui code pour un/le répresseur (ArgR) de la biosynthèse de la L-arginine,
h) le gène argF, qui code pour une ornithine carbamoyltransférase (ArgF, EC 2.1.3.3),
i) le gène argG, qui code pour une argininosuccinate synthase (ArgG, EC 6.3.4.5),
j) le gène argH, qui code pour une argininosuccinate lyase (ASAL) (ArgH, EC 4.3.2.1),
k) le gène lysC, qui code pour une aspartatekinase (LysC, EC 2.7.2.4), et
l) le gène asd, qui code pour une aspartatesemialdéhyde-déshydrogénase (Asd, EC 1.2.1.11).

6. Procédé selon les revendications 1 à 5, **caractérisé en ce qu'**on renforce en outre un ou plusieurs des gènes choisis dans le groupe
a) glutamate-déshydrogénase (EC 1.4.1.3) codée par le gène gdh,
b) glutamate N-acétyltransférase (EC 2.3.1.35 et EC 2.3.1.1) codée par le gène argJ,
c) acétylglutamatekinase (EC 2.7.2.8) codée par le gène argB,
d) N-acétyl-gamma-glutamyl-phosphate-réductase (EC 1.2.1.38) codée par le gène argC,
e) acétyl-ornithine aminotransférase (EC 2.6.1.11), codée par le gène argD,
f) composant EIIB spécifique du glucose (PtsG) (EC 2.7.1.69) du système d'absorption du glucose codé par le gène ptsG,
g) composant EIIB spécifique du saccharose (PtsS) (EC 2.7.1.69) du système d'absorption du saccharose codé par le gène ptsS,
h) glucose-6-phosphate 1-déshydrogénase (EC 1.1.1.49) codée par le gène zwf,
i) glucose-6-phosphate-isomérase (EC 5.3.1.9) codée par le gène pgi,
j) phosphofructokinase (EC 2.7.1.11) codée par le gène pfkA,
k) fructose-bisphosphate aldolase (EC 4.1.2.13) codée par le gène fda,
l) glycéraldéhyde-3-phosphate déshydrogénase (EC 1.2.1.59), codée par le gène gap,
m) phosphoglycérate kinase (EC 2.7.2.3) codée par le gène pgk,
n) pyruvatekinase (EC 2.7.1.40) codée par le gène pyk,
o) sous-unité E1 de la pyruvate-déshydrogénase (EC 1.2.4.1) codée par le gène aceE,
p) phosphoénolpyruvate-carboxylase (EC 4.1.1.31) codée par le gène ppc,
q) pyruvate-carboxylase (EC 6.4.1.1), codée par le gène pyc,
r) aconitase (EC 4.2.1.3) codée par le gène acn, et
s) isocitrate-déshydrogénase (EC 1.1.1.42) codée par le gène icd.

7. Procédé selon les revendications 1 à 6, **caractérisé en ce qu'**il s'agit d'un procédé choisi dans le groupe procédé discontinu, procédé discontinu alimenté, procédé discontinu alimenté répété et procédé continu.

8. Procédé selon les revendications 1 à 7, **caractérisé en ce qu'**on obtient à partir du bouillon de fermentation contenant de la L-ornithine la L-ornithine ou un produit solide ou liquide contenant la L-ornithine.
